# EUROPEAN PATENT SPECIFICATION

(11) **EP 0 589 841 B1**
(45) Date of publication and mention of the grant of the patent: **04.05.2005**
(21) Application number: 93810654.9
(22) Date of filing: 15.09.1993
(51) Int. Cl.: C12N 15/82, C12N 5/10, A01H 1/02, A01H 5/00

(54) **Methods for the production of hybrid seed**
Verfahren zur Herstellung von hybridem Saatgut
Procédés de production de semences hybrides

(30) Priority: 24.09.1992 US 950348
(43) Date of publication of application: 30.03.1994
(73) Proprietor: Syngenta Participations AG, 4058 Basel (CH)
(72) Inventor: Crossland, Lyle D., Chapel Hill, NC 27514 (US); Tuttle, Annmarie, Garner, NC 27529 (US); Stein, Jeffrey I., Hillsborough NC 27278 (US)

(56) References cited:
- EP-A- 0 412 006
- WO-A-90/08830
- WO-A-90/13654
- WO-A-91/03561
- WO-A-91/13994
- WO-A-92/01799
- WO-A-92/04454
- US-A- 4 990 607
- PLANT MOLECULAR BIOLOGY, vol.17, 1991 pages 229 - 234 LASSNER, M.W., ET AL. 'Targeting of T7 RNA polymerase to tobacco nuclei mediated by an SV40 nuclear location signal'
- THE PLANT CELL, vol.2, no.12, December 1990 pages 1201 - 1204 KOLTUNOW, A.M., ET AL. 'Different temporal and spatial gene expression patterns occur during anther development'
- NATURE, vol.334, 1988 pages 631 - 633 MA, J., ET AL. 'Yeast activators stimulate plant gene expression'

## Description

The present invention is in the field of plant genetic engineering. In particular, it relates to a method for the production of male-sterile plants and the use of such plants in producing hybrid seed. It further relates to transgenic male-sterile plants and hybrid seed produced from the method according to the invention.

Heterosis in corn has received considerable attention because of its marked effect on yield improvement. This increased productivity on crossing different strains of corn was first noted in the late 19th century and was then developed according to systematic genetic procedures.

The usual method for raising hybrid corn is to establish many inbred lines, make intercrosses, and determine which hybrids are more productive in a given locality.

The success of hybrid maize motivated plant breeders to explore the existence and magnitude of hybrid vigor in many other species with economic importance. In general, hybrids increase yields. They are usually more efficient in use of growth factors and give a greater return per unit for the growth factors such as water and fertilizer. Under stress F1 hybrids are generally superior to parental cultivars, with a more stable performance over a wide range of environments. With hybrids, there is uniformity in product and maturity that often facilitates harvest and increases the value of the product in the marketplace. The F1 hybrid may combine characters that are difficult or impossible to combine in other ways. This is particularly true of many interspecific and intergeneric hybrids. The general conclusion from research is that hybrid vigor, a common phenomenon in plants is of sufficient magnitude to warrant commercial exploitation if appropriate techniques can be devised.

Hybrid vigor has been recognized as a wide-spread phenomenon in plants and animals for many years. Commercial hybrids are now used extensively in many crops, including corn, sorghum, sugarbeet, and sunflower. Research is being conducted on many other crops that may permit the wide-spread use of commercial hybrids in the future.

Commercial hybrids have the greatest potential for crops in which the hybrid seed can be produced reliably and economically. Three biological requirements for successful hybrid seed production include the presence of hybrid vigor, elimination of fertile pollen in the female parent, and adequate pollination by the male parent.

In order to produce hybrid seed uncontaminated with selfed seed, pollination control methods must be implemented to ensure cross-pollination and not self-pollination. Known pollination control mechanisms are generally mechanical, chemical, or genetic.

Elimination of fertile pollen from the female parent can be achieved by hand emasculation in some species such as maize, a monoecious species. Such elimination of fertile pollen is achieved by pulling or cutting the male inflorescence (tassel) from plants in the female parent population. This simple procedure prevents self-fertilization by mechanically detasseling female plants before pollen shed to prevent selfing. However, most major crop plants of interest have both functional male and female organs within the same flower. Thus, emasculation is not a simple procedure. At any rate, this form of hybrid seed production is extremely labor intensive and hence expensive.

To eliminate the laborious detasseling that is necessary to prevent self-fertilization in hybrid crosses, cytoplasmic factors which produce male-sterility have been used in some species in conjunction with restorer genes.

Male-sterility in the female parent can be controlled by nuclear genes or by a cytoplasmic-genetic system. Genetic male-sterility is controlled by nuclear genes in which the alleles for sterility generally are recessive to the alleles for fertility. Genetic male-sterility occurs in many species. Usually, it is controlled by a single recessive gene that must be homozygous to cause male-sterility. Breeders who use genetic male-sterility for hybrid seed production usually develop a phenotypically uniform female line that segregates 1:1 for Msms and no Msms individuals. Seed for these lines is increased in isolation by harvesting seed from msms plants that are pollinated from Msms plants. To produce commercial F1 hybrid seed with genetic male-sterility, the 50 percent of female Msms plants must be rouged from the field as soon as their fertility can be identified. The labor associated with rouging fertile plants from female plants has greatly restricted the use of genetic male-sterility in producing hybrid seed. There are several problems associated with this system for producing commercial hybrid seed. First, it is not possible to eliminate fertile plants from the desired male-sterile plants in the female population. Genetic male-sterile plants must be maintained by mating them with male-fertile individuals. Half of the F1 plants from such a cross would be sterile, but the remaining plants would be fertile. Thus, the unwanted male-fertile plants in the female population may disseminate pollen and reduce the effectiveness of the desired male parent.

The successful use of cytoplasmic male-sterility for commercial hybrid seed production requires a stable male-sterile cytoplasm, an adequate pollen source, and an effective system of getting the pollen from the male parent to the male-sterile female. Also, the cytoplasmic-genetic system of male sterility requires three lines to produce a single crossed hybrid; the A line (male-sterile), B line (male-fertile maintainer), and R line (male-fertile with restorer genes). Three-way crosses produced with cytoplasmic-genetic male sterility involved maintenance and production of four lines, an A and B line of one inbred and male-fertile inbreds of the other two.

Furthermore, the southern corn blight caused by Helminthosporium maydis, Race T, which severely attacked all maize hybrids with cytoplasmic male-sterile T cytoplasm, demonstrated the vulnerability of a hybrid seed production industry based on a single source of male-sterile cytoplasm. For hybrid maize, most seed producers have returned to hand or mechanical emasculation and wind pollination.

Hybrid seed may also be produced by the use of chemicals that block or kill viable pollen formation. These chemicals, gametocides, are used to impart a transitory male-sterility. However, the expense and availability of the chemicals and the reliability of the applications limits the production of hybrid seed by using gametocides.

Molecular methods for hybrid seed production have also been described. Such methods transform plants with constructs containing anti-sense DNA and other genes which are capable of controlling the production of fertile pollen into plants. Such regenerated plants are functionally male-sterile and are used for the production of hybrid seed by crossing with pollen from male-fertile plants. The primary deficiencies of these approaches stem from the fact that the genetically engineered male sterility gene, whether it is an anti-sense or RNAse, can only be maintained in a heterozygous state. They are fundamentally the same as natural genetic male steriles in that they must be maintained by crossing to isogenic male fertile lines. This is most problematic in the hybrid cross field where the acreage is large and yield is critical. The heterozygous female parent, of which only 50% will be male sterile, must be planted in rows next to the pollen donor male parent and the 50% fertile female parents removed. This is rendered easier in genetically engineered genetic male steriles because a herbicide resistance gene can be linked to the male sterility gene, and herbicide spray can be used to remove the fertile plants, but it still means that the female parent rows must be planted at double density in order to get the same yield per acre of our system. This will cause some yield loss due to competition. The herbicide spray also means yield loss because the resistant plants are never 100% immune to the herbicide, and the costs of spraying the chemical are considerable.

Accordingly, it was the main object of the present invention to provide a reliable simple technique for the formation of hybrid seed production that does not involve the disadvantages of the known methods as described before.

This object could be achieved within the scope of the present invention by providing a method for producing male-sterile plants. The method according to the invention comprises crossing two genetically transformed plants, parents 1 and 2. Parent 1 is transformed with an expression cassette which comprises a nucleotide sequence which encodes a first polypeptide, a transactivator, capable of regulating a second nucleotide sequence, a target nucleotide sequence. The DNA sequence encoding the first polypeptide is operably linked to an anther specific promoter.

Parent 2 is transformed with an expression cassette which comprises the target nucleotide sequence operably linked to a nucleotide sequence which encodes RNA or a polypeptide, both of which are capable of disrupting the formation of viable pollen. When parents 1 and 2 are crossed, polypeptide 1, the transactivator, regulates the target nucleotide sequence and turns on the expression of polypeptide 2. Thus, no viable pollen is formed in the subsequent generation.

Accordingly, the present invention primarily relates to a method for producing male-sterile plants, said method comprising:
(i) transforming a first parent plant cell with a first expression cassette, said cassette comprising a nucleotide sequence encoding an anther-specific 5' regulatory region operably linked to a nucleotide sequence which encodes a transactivator polypeptide; and regenerating a transformed plant, Parent 1, from said first transformed plant cell;
(ii) transforming a second parent plant cell with a second expression cassette comprising a target nucleotide sequence which is capable of being activated by said transactivator polypeptide operably linked to a nucleotide sequence which encodes anti-sense RNA or a polypeptide capable of disrupting the formation of viable pollen; and
   regenerating a transformed plant, Parent 2, from said second transformed plant cell;
(iii) crossing said parent 1 with said parent 2 to obtain male-sterile offspring.

The invention thus further relates to transgenic plants and the progeny thereof, which comprises a stably integrated expression cassette wherein said expression cassette comprises a nucleotide sequence encoding an anther-specific promoter which is operably linked to a nucleotide sequence encoding a transactivator.

The invention also relates to transgenic plants and the progeny thereof, which comprise a stably integrated expression cassette wherein said expression cassette comprises a target nucleic acid sequence, which is capable of being activated by a transactivator, operably linked to a nucleotide sequence which encodes anti-sense RNA or a polypeptide which will disrupt the formation of viable pollen.

Further comprised are transgenic male-sterile plants and the progeny thereof, which comprise a stably integrated first expression cassette comprising a nucleotide sequence encoding an anther-specific promoter which is operably linked to a nucleotide sequence encoding a transactivator and a second expression cassette comprising a target nucleotide sequence, which is activated by said transactivator polypeptide, operably linked to a nucleotide sequence which encodes anti-sense RNA or a polypeptide which will disrupt the formation of viable pollen.

The transgenic plant according to the invention may be a dicotyledonous or a monocotyledonous plant. Preferred are monocotyledonous plants of the Graminaceae family involving Lolium, Zea, Triticum, Triticale, Sorghum, Saccharum, Bromus, Oryzae, Avena, Hordeum, Secale and Setaria plants.

Especially preferred are transgenic maize, wheat and barley plants.

The expression 'progeny' is understood to embrace both, "asexually" and "sexually" generated progeny of transgenic plants. This definition is also meant to include all mutants and variants obtainable by means of known processes, such as for example cell fusion or mutant selection and which still exhibit the characteristic properties of the initial transformed plant, together with all crossing and fusion products of the transformed plant material.

Another object of the invention concerns the proliferation material of transgenic plants.

The proliferation material of transgenic plants is defined relative to the invention as any plant material that may be propagated sexually or asexually in vivo or in vitro. Particularly preferred within the scope of the present invention are protoplasts, cells, calli, tissues, organs, seeds, embryos, pollen, egg cells, zygotes, together with any other propagating material obtained from transgenic plants.

Parts of plants, such as for example flowers, stems, fruits, leaves, roots originating in transgenic plants or their progeny previously transformed by means of the process of the invention and therefore consisting at least in part of transgenic cells, are also an object of the present invention.

The male-sterile plants according to the invention are useful for producing hybrid seed, which is a further object of the invention.

Accordingly, the invention further relates to a method for producing hybrid seed from plants selected from those species of pollen producing plants which are capable of being genetically transformed, said method comprising:
(a) producing male-sterile plants by:
   (i) transforming a first parent plant cell with a first expression cassette which comprises a nucleotide sequence encoding an anther-specific 5' regulatory region which is operably linked to a nucleotide sequence encoding a transactivator;
   (ii) regenerating a transformed plant, Parent 1, from said first transformed plant cell;
   (iii) transforming a second parent plant cell with an expression cassette which comprises a target nucleotide sequence which is activated by said transactivator operably linked to a nucleotide sequence which encodes anti-sense RNA or a polypeptide which will disrupt the formation of viable pollen;
   (iv) regenerating a transformed plant, Parent 2, from said second transformed plant cell; and (v) crossing said parent 1 with said parent 2 to produce male-sterile offspring; and
(b) crossing said male-sterile offspring with a selected fertile line to obtain hybrid seed.

Also comprised by the invention are thus hybrid seed produced from a method as described before.

The invention thus further comprises transgenic plants that have been transformed with both of the above constructs. The transgenic plant according to the invention may be a dicotyledonous or a monocotyledonous plant. Preferred are monocotyledonous plants of the Graminaceae family involving Lolium, Zea, Triticum, Triticale, Sorghum, Saccharum, Bromus, Oryzae, Avena, Hordeum, Secale and Setaria plants.
Especially preferred are transgenic maize, wheat and barley plants.

In particular, the present invention provides a dual system for production of male-sterile plants. The system involves crossing two genetically transformed plants, herein referred to as parents 1 and 2. Parent 1 is transformed with an expression cassette which comprises a nucleotide sequence which directs the expression of a first polypeptide in anthers. This first polypeptide is capable of regulating the transcription of a second nucleotide sequence, the target DNA, which directs expression of RNA or a second polypeptide capable of disrupting the production of viable pollen. Parent 2 is transformed with an expression cassette which comprises the target nucleotide sequence operably linked to a nucleotide sequence which encodes RNA or a polypeptide capable of disrupting the formation of viable pollen.

As noted, the RNA or the second polypeptide can only be expressed when in the presence of the first polypeptide, the transactivator. Thus, both parents 1 and 2 are male-fertile. However, upon crossing parent 1 with parent 2, the transactivator regulates the expression of the RNA or polypeptide 2 via the target DNA sequence. The result is no viable pollen is produced. The resulting progeny containing both expression cassettes are male sterile.

Male sterility is the failure or inability to produce functional or viable pollen. Male sterility may result from defects leading to the non-formation of pollen or to the lack of functional ability in the pollen when it is formed. Therefore, either pollen is not formed or, if formed, it is either non-viable or incapable of effective fertilization under normal conditions.

The male-sterile plants of the invention, are female fertile. That is, the plants do not produce fertile pollen, yet are capable of accepting pollen from the desired paternal parent resulting in fertilization and seed production.

There are several transactivator polypeptides which can be used in the present dual sterility system. The important aspect is that the RNA or the second polypeptide which disrupts pollen formation is not expressed in the absence of the first or transactivator polypeptide.

The transactivators of the invention are capable of activating a target nucleotide sequence which is operably linked to a nucleotide sequence which encodes RNA or a second polypeptide both of which are capable of disrupting the production of viable pollen. Thus, the nucleotide sequence operably linked to the target sequence is only expressed in the presence of the transactivator.

The transactivators of the invention include, but are not limited to, polymerases, DNA binding proteins, naturally occurring and synthetic transcriptional activators, translational activators, post-transcriptional activators, and the like. The use of such transactivator polypeptides in directing expression of another nucleotide sequence is exemplified by the T7 RNA polymerase. See, U.S. Patent Numbers 5,122,457; 5,126,251; and 5,135,855; Lassner et al., (1991) Plant Molecular Biology 17:229-234; Rodriguez et al., (1990) Journal of Virology 64:4851-4857; Vennema et al., (1991) Gene 108:201-210; Benton et al., Molecular and Cellular Biology (1990) Molecular and Cellular Biology 10:353-360; Elroy-Stein and Moss (1990) Proceedings, Proc. Natl. Acad. Sci.:USA 87:6743-6747; Moss et al., (1990) Nature 348:91-92; Elroy-Stein et al., (1989) Proceedings, Proc. Natl. Acad. Sci.:USA 86:6126-6130; and Rosenberg et al., (1987) Gene 56:125-135.

Regulator polypeptides or transactivators also include DNA binding proteins which are necessary for transcription activation of specific promoters. Binding domains of one protein may be fused to activity domains of another protein to form chimeras of such DNA binding proteins, such as GAL4/VP16 (Carey et al. (1989), J. Mol. Biol., 209:423-432; Cress et al. (1991) Science, 251:87-90; and Sadowski et al. (1988), Nature, 335:563-564). Likewise, the binding domain of other proteins, i.e., Lex A (Brent and Ptashne, (1985), Cell, 43:729-736, which describes a Lex A/GAL4 transcriptional activator) can be utilized.

Translational activators are exemplified by the cauliflower mosaic virus translational activator (TAV). See, for example Fuetterer and Hohn (1991) EMBO J. 10:3887-3896. In this system a dicistronic mRNA is produced. That is, two coding regions are transcribed in the same mRNA from the same promoter. In the absence of TAV, only the first cistron is translated by the ribosomes. However, in cells expressing TAV, both cistrons are translated. The coding region for a polypeptide capable of disrupting the formation of viable pollen is placed in the position of the second cistron.

The expression cassette, expression cassette 1, utilized to transform parent 1 comprises an anther 5' regulatory region operably linked to the first polypeptide, the transactivator. The 5' regulatory regions of the invention include nucleotide sequences necessary for expression, i.e. the promoter region. The construct may also include any other necessary regulators such as terminators,(Guerineau et al., (1991), Mol. Gen. Genet., 226:141-144; Proudfoot, (1991), Cell, 64:671-674; Sanfacon et al., (1991), Genes Dev., 5:141-149; Mogen et al., (1990), Plant Cell, 2:1261-1272; Munroe et al., (1990), Gene, 91:151-158; Ballas et al., (1989), Nucleic Acids Res., 17:7891-7903; Joshi et al., (1987), Nucleic Acid Res., 15:9627-9639); nuclear localization signals (Kalderon et al., (1984) Cell, 39:499-509; and Lassner et al., (1991) Plant Molecular Biology, 17:229-234); plant translational consensus sequences (Joshi, C.P., (1987), Nucleic Acids Research, 15:6643-6653), introns (Luehrsen and Walbot, (1991), Mol. Gen. Genet., 225:81-93) and the like, operably linked to the nucleotide sequence of the transactivator.

The expression cassette, expression cassette 2, utilized to transform parent 2 comprises a nucleotide sequence upon which the transactivator acts operably linked to a coding region of interest. Additional regulating nucleotide regions may also be included, such as terminators, promoters, leader sequences and the like. Such regions are operably linked to the coding region.

It may be beneficial to include 5' leader sequences in the expression cassette 2 construct. Such leader sequences can act to enhance translation. Translational leaders are known in the art and include:
Picornavirus leaders, for example, EMCV leader (Encephalomyocarditis 5' noncoding region) (Elroy-Stein, O., Fuerst, T.R., and Moss, B. (1989) PNAS USA 86:6126-6130);
Potyvirus leaders, for example, TEV leader (Tobacco Etch Virus) (Allison et al., (1986); MDMV leader (Maize Dwarf Mosaic Virus); Virology, 154:9-20), and
Human immunoglobulin heavy-chain binding protein (BiP), (Macejak, D.G., and Sarnow, P., (1991), Nature, 353:90-94;
untranslated leader from the coat protein mRNA of alfalfa mosaic virus (AMV RNA 4), (Jobling, S.A., and Gehrke, L., (1987), Nature, 325:622-625;
Tobacco mosaic virus leader (TMV), (Gallie, D.R. et al., (1989), Molecular Biology of RNA, pages 237-256; and
Maize Chlorotic Mottle Virus leader (MCMV) (Lommel, S.A. et al., (1991), Virology, 81:382-385. See also, Della-Cioppa et al., (1987), Plant Physiology, 84:965-968.

Either a plant terminator, a T7 terminator or both may be utilized in expression cassette 2. See, Rosenberg et al., (1987), Gene, 56:125; Guerineau et al., (1991), Mol. Gen. Genet., 226:141-144; Proudfoot, (1991), Cell, 64:671-674; Sanfacon et al., (1991), Genes Dev., 5:141-149; Mogen et al., (1990), Plant Cell, 2:1261-1272; Munroe et al., (1990), Gene, 91:151-158; Ballas et al., (1989), Nucleic Acids Res., 17:7891-7903; Joshi et al., (1987), Nucleic Acid Res., 15:9627-9639.

Particular expression cassettes will be discussed in more detail for different transactivator systems and exemplified in the experimental section.

In one embodiment of the invention, a T7 polymerase system is utilized. The T7 bacteriophage harbours a gene coding for an RNA polymerase which recognizes a phage specific promoter. The polymerase and the phage promoters have unique properties which prevent interference with expression of host genes.

The T7 RNA polymerase is a monomeric enzyme of 100 kD whereas most other polymerases are more complex. The T7 promoter consists of 23 bp which are not encountered in other prokaryotic or eukaryotic promoters. See, Dunn et al., (1983) J. Mol. Biol. 166:477-535; Davanloo et al., (1984) Proceedings Proc. Natl. Acad. Sci.:USA 81:2035-2079; and Moffatt et al., (1984) J. Mol. Biol. 173:265-269.

To use a T7 polymerase system to produce male-sterile plants, parent 1 is transformed with an expression cassette comprising an anther 5' regulatory region operably linked to a nucleotide sequence encoding the T7 polymerase. A nuclear location signal (NLS) such as the SV40 nuclear location signal may also be incorporated into the construct. See, for example, Kalderon et al., (1984) Cell 39:499-509; Dunn et al., (1988) Gene 68:259-266; Hunt T. (1989) Cell 59:949-951; and Lassner et al., (1991) Plant Molecular Biology 17:229-234. A plant translational consensus sequence (Joshi, C.P. (1987) Nucleic Acids Research 15:6643-6653) may be also included, as well as plant termination signals and introns.

Anther-specific promoters are known in the art. By utilizing anther-specific promoters, the resulting transgenic plants will express the T7 polymerase only in the anther of the plant. Anther-specific promoters are set forth in the European Patent Application Number 93810455.1 filed June 24, 1993.

Anther-specific genomic clones may be obtained by using anther-specific cDNA clones as probes to pull out the corresponding genomic DNA clones. Corresponding genomic DNA clones are those which are transcribed to form a messenger RNA which is complementary to and transcribed into a given cDNA.

The anther-specific cDNA sequence may be obtained by preparing cDNA libraries from anther tissue and leaf tissue. Single stranded DNA from the leaf is photobiotinylated and hybridized to the anther DNA. Photobiotinylated DNA is removed, leaving a library enriched for anther-specific cDNA sequences. Anther-specific cDNAs are identified by differential screening. The anther-specific cDNAs are cross-hybridized to identify unique cDNAs. Anther-specific expression is verified by RNA blot hybridization with various plant tissues and *in situ* hybridization. Developmental expression, sequences and gene copy number of the anther-specific cDNA clones may also be determined.

The cDNA sequences can be used to isolate genomic DNA sequences. Where a partial cDNA has been obtained, the partial cDNA is used as a probe to screen the anther cDNA library in order to isolate a full length cDNA clone. Hybridizing clones are purified, restriction mapped and sequenced. A full length clone will be near message size as well as having a complete open reading frame. To isolate a genomic clone, the full length anther cDNA is used as a probe to screen a genomic library. By restriction mapping and hybridization to the anther cDNA, the coding region of the genomic clone is identified. The area upstream from the coding area of the clone is the anther promoter region.

The anther-specific promoter region may be more precisely mapped through deletion analysis. 5' deletions of an anther promoter are made by introducing restriction sites by PCR using oligonucleotide primers with restriction sites at the 5' ends and anther promoter sequences at the 3' ends. The PCR products are digested, purified, and cloned into a suitable plasmid vector such as, for example, pBI101 (Clontech). The deletion mutants contain the 5' untranslated leader sequence fused to the translational start site of the GUS gene. Internal and 3' deletions of anther promoters are made by PCR in a similar manner. The PCR fragments are fused to a GUS vector containing the CAMV 35S minimal promoter [-46 to +1; Benfey *et al*, 1990]. Transgenic plants can then be tested with the GUS fluorometric and histochemical assay.

In the case of promoter DNA sequences, "anther-specific" describes regulatory sequences which direct the transcription of associated coding sequences so that the corresponding messenger RNA is present in anther tissue in concentrations at least about 100-fold that observed in other tissues.

The expression cassette utilized to transform parent 2 comprises a T7 promoter operably linked to a nucleotide sequence which encodes RNA or a polypeptide which disrupts formation of viable pollen when expressed. Such polypeptides include but are not limited to:
Diphtheria Toxin A-chain (DTA), which inhibits protein synthesis, Greenfield et al., (1983), Proc. Natl. Acad., Sci.:USA, 80:6853; Palmiter et al., (1987), Cell, 50:435;
Pectate lyase pelE from Erwinia chrysanthemi EC16, which degrades pectin, causing cell lysis. Keen et al., (1986), J. Bacteriology, 168:595;
T-urf13 (TURF-13) from cms-T maize mitochondrial genomes; this gene encodes a polypeptide designated URF13 which disrupts mitochondrial or plasma membranes. Braun et al., (1990), Plant Cell, 2:153; Dewey et al., (1987), Proc. Natl. Acad. Sci.:USA, 84:5374; Dewey et al., (1986), Cell, 44:439;
Gin recombinase from phage Mu a gene, which encodes a site-specific DNA recombinase which will cause genome rearrangements and loss of cell viability when expressed in cells of plants. Maeser et al., (1991), Mol. Gen. Genet., 230:170-176;
Indole acetic acid-lysine synthetase (iaaL) from Pseudomonas syringae, which encodes an enzyme that conjugates lysine to indoleacetic acid (IAA). When expressed in the cells of plants, it causes altered developments due to the removal of IAA from the cell via conjugation. Romano et al., (1991), Genes and Development, 5:438-446; Spena et al., Mol. Gen. Genet., (1991), 227:205-212; Roberto et al., Proc. Natl. Acad. Sci.:USA, 87:5795-5801; and,
CytA toxin gene from Bacillus thuringiensis Israeliensis which encodes a protein that is mosquitocidal and hemolytic. When expressed in plant cells, it causes death of the cell due to disruption of the cell membrane. McLean et al., (1987), J. Bacteriology, 169:1017-1023; Ellar et al., (1990), United States Patent No. 4,918,006.

Such polypeptides also include Adenine Phosphoribosyltransferase (APRT) (Moffatt and Somerville, (1988), Plant Physiol., 86:1150-1154); DNAse, RNAse; protease; salicylate hydroxylase; etc.

It is further recognized that the T7 promoter could be operably linked to RNA which is capable of disrupting the formation of viable pollen. The RNA of the invention includes antisense RNA as well as ribozymes. Antisense RNA can be utilized which will hybridize with mRNA from a gene which is critical to pollen formation or function, i.e. APRT. In this manner, the anti-sense RNA will prevent expression of the necessary genes resulting in no pollen formation.

Alternately, ribozymes can be utilized which target mRNA from a gene which is critical to pollen formation or function. Such ribozymes will comprise a hybridizing region of at least about nine nucleotides which is complementary in nucleotide sequence to at least part of the target RNA and a catalytic region which is adapted to cleave the target RNA. Ribozymes are described in EPA No. 0 321 201 and WO88/04300. See, also Haseloff and Gerlach, (1988), Nature, 334:585-591; Fedor and Uhlenbeck, (1990), Proc. Natl. Acad. Sci.: USA, 87:1668-1672; Cech and Bass, (1986), Ann. Rev. Biochem., 55:599-629; Cech, T.R., (1987), 236:1532-1539; Cech, T.R. (1988) Gene, 73:259-271; and, Zang and Cech, (1986), Science, 231:470-475.

When parent 1 is crossed with parent 2, the progeny contain both expression cassettes. Therefore, the T7 polymerase drives expression of the coding sequence under the control of the T7 promoter. A polypeptide or RNA is expressed which disrupts the formation of viable pollen resulting in male-sterility.

Having described the T7 polymerase system in detail, one of skill in the art will recognize that other transactivators may be utilized to obtain the same effect.

Other known transactivators include, but are not limited to GAL4 (Carey et al., (1989), J. Mol. Biol., 209:423-432; Ginger et al., (1985), Cell, 40:767-774); VP16 (Cress and Triezenberg (1991), Science, 251:87-90); GAL4-VP16 (Sadowski et al., (1988), Nature, 335:563-564); etc. See also, Ma and Ptashne, (1987), Cell, 43:729-736; Hope and Struhl, (1986), Cell, 46:885-894; and Gill and Ptashne, (1987), Cell, 51:121-126. Such transactivators activate transcription in yeast, plant, insect and mammalian cells. These proteins typically contain two parts. One part directs DNA binding and the other, the activating region, presumably interacts with some component of the transcriptional machinery. Thus, fusions such as GAL4-VP16; GAL4-c1 may be utilized.

The transactivators GAL4/VP16 and GAL4/c1 can be utilized to transactivate a promoter with at least one GAL4 binding site. In this system, parent 1 is transformed with an expression cassette comprising an anther 5' regulatory region operably linked to GAL4/VP16 or GAL4/c1. Parent 2 is transformed with an expression cassette comprising in 5' to 3' orientation, a GAL4 binding site, a minimal promoter or 5' regulatory region and the coding region of interest. By minimal promoter is intended that the basal promoter elements are inactive or nearly so without upstream activation.

The offspring from a cross of parent 1 and parent 2 will be male-sterile as the GAL4 transactivator will direct expression of the polypeptide or anti-sense RNA which will disrupt formation of viable pollen.

As discussed earlier, translational activators can also be utilized. In this system, Parent 1 is transformed with an expression cassette comprising an anther 5' regulatory region operably linked to the cauliflower mosiac virus translation activator (TAV). Parent 2 is transformed with an expression cassette comprising an anther specific promoter operably linked to a dicistronic mRNA wherein the second cistron encodes a cell toxin. Crossing parents 1 and 2 results in male sterile offspring as both cistrons of the dicistronic mRNA will be translated in the presence of TAV. See, Bonneville et al., (1987), Cell, 59:1135-1143; Fuetterer and Hohn, (1991), EMBO J., 10:3887-3896; Gowda et al., (1988), Proc. Natl. Acad, Sci., USA, 86:9203-9207; Scholthof et al., (1992), J. Virology, 66:3131-3139; and EP 298 918 filed July 10, 1987.

In some instances it may be useful to combine the use of more than one transactivator in a single system. For example, transcriptional activation via T7 polymerase or GAL4/VP16 can be combined with translational activation. This combination may provide a tighter control of unwanted expression of the toxin gene in the absence of the transactivator.

Methodologies for the construction of plant expression cassettes as well as the introduction of foreign DNA in to plants is generally described in the art. Generally, for the introduction of foreign DNA into plants Ti plasmid vectors have been utilized for the delivery of foreign DNA as well as direct DNA uptake, liposomes, electroporation, micro-injection, and the use of microprojectiles. Such methods had been published in the art. See, for example, Guerche et al., (1987) Plant Science 52:111-116; Neuhaus et al., (1987) Theor. Appl. Genet. 75:30-36; Klein et al., (1987) Nature 327: 70-73; Howell et al., (1980) Science 208:1265; Horsch et al., (1985) Science 227: 1229-1231; DeBlock et al., (1989) Plant Physiology 91:694-701; Methods for Plant Molecular Biology (Weissbach and Weissbach, eds.) Academic Press, Inc. (1988); and Methods in Plant Molecular Biology (Schuler and Zielinski, eds.) Academic Press, Inc. (1989). It is understood that the method of transformation will depend upon the plant cell to be transformed.

One possible method for introducing genetic material into plant cells comprises, for example, bringing plant cells into contact with viruses or with Agrobacterium comprising the DNA to be introduced. This may be achieved by infecting sensitive plant cells or by co-cultivating protoplasts derived from plant cells. Within the scope of this invention, Cauliflower Mosaic Virus (CaMV) may also be used as a vector for the insertion of the DNA constructs according to the invention into a plant.

Another method makes use of the infection of the plant cell with Agrobacterium tumefaciens and/or Agrobacterium rhizogenes, which has previously been transformed with the said gene construction. The transgenic plant cells are then cultured under suitable culture conditions known to the person skilled in the art, so that they form shoots and roots and whole plants are finally formed.

A further possible method of transforming plant material comprises mixed infection using both Agrobacterium rhizogenes and transformed Agrobacterium tumefaciens, as described by Petit et al, (1986), Mol Gen Genet 202: 388 for the transformation of carrots.

The plant expression cassettes according to the invention can therefore be transferred into suitable plant cells by means of, for example, the Ti-plasmid of Agrobacterium tumefaciens or the Ri-plasmid of Agrobacterium rhizogenes. The Ti-plasmid or Ri-plasmid is transferred to the plant in the course of infection by Agrobacterium and integrated in stable manner into the plant genome.

Any T-DNA-containing vector that can be transferred into plant cells and permits selection of the transformed cells is suitable for use within the scope of this invention such as, for example, a shuttle vector that comprises the DNA constructs according to the invention cloned in between the left border sequence (LB) and the right border sequence (RB) and that is capable of stable replication both in E. coli and in A. tumefaciens. Preferred is a so-called binary vector system.

Using newly developed transformation techniques, it has also become possible in principle to transform in vitro plant species that are not natural host plants for Agrobacterium. For example, monocotyledonous plants, especially the cereal species and various grasses, are not natural hosts for Agrobacterium.

It has become increasingly evident that monocotyledons can also be transformed using Agrobacterium, so that, using new experimental formulations that are now becoming available, cereals and grass species are also amenable to transformation (Grimsley et al, EP-A-0 267 159).

One of the preferred methods for introducing DNA into a plant cell by means of Agrobacterium is the so-called leaf disk transformation using Agrobacterium (Horsch et al., (1985) Science 227: 1229-1231). Sterile leaf disks from a suitable target plant are incubated with Agrobacterium cells comprising one or more of the expression cassettes according to the invention, and are then transferred into or onto a suitable nutrient medium. Especially suitable, and therefore preferred within the scope of this invention, are LS media that have been solidified by the addition of agar and enriched with one or more of the plant growth regulators customarily used, especially those selected from the group of the auxins consisting of a-naphthylacetic acid, picloram, 2,4,5-trichlorophenoxyacetic acid, 2,4-dichlorophenoxyacetic acid, indole-3-butyric acid, indole-3-lactic acid, indole-3-succinic acid, indole-3-acetic acid and p-chlorophenoxyacetic acid, and from the group of the cytokinins consisting of kinetin, 6-benzyladenine, 2-isopentenyladenine and zeatin. The preferred concentration of auxins and cytokinins is in the range of from 0.1 mg/l to 10 mg/l.

After incubation for several days but preferably after incubation for 2 to 3 days at a temperature of from 20C to 40C, preferably from 23C to 35C and more especially at 25C and in diffuse light, the leaf disks are transferred to a suitable medium for the purpose of shoot induction. Especially preferred for the selection of the transformants is an LS medium that does not contain auxin but contains cytokinin instead, and to which a selective substance has been added dependent on the marker gene used. The cultures are kept in the light and are transferred to fresh medium at suitable intervals, but preferably at intervals of one week. Developing green shoots are cut out and cultured further in a medium that induces the shoots to form roots. Especially preferred within the scope of this invention is an LS medium that does not contain auxin or cytokinin but to which a selective substance has been added for the selection of the transformants.

In addition to Agrobacterium-mediated transformation, within the scope of this invention it is possible to use direct transformation methods for the insertion of the gene constructions according to the invention into plant material.

Possible methods for the direct transfer of genetic material into a plant cell comprise, for example, the treatment of protoplasts using procedures that modify the plasma membrane, for example, polyethylene glycol treatment, heat shock treatment or electroporation, or a combination of those procedures Shillito et al, (1985), Bio Technology, 3: 1099-1103.

In the electroporation technique, plant protoplasts together with plasmids that comprise the expression cassettes according to the present invention are subjected to electrical pulses of high field strength. This results in a reversible increase in the permeability of biomembranes and thus allows the insertion of the plasmids. Electroporated plant protoplasts renew their cell wall, divide and form callus tissue. Selection of the transformed plant cells can take place with the aid of the above-described phenotypic markers.

A further method for the direct introduction of genetic material into plant cells, which is based on purely chemical procedures and which enables the transformation to be carried out very efficiently and rapidly, is described in Negrutiu et al, (1987), Mol Gen Genet 199: 330-337.

Also suitable for the transformation of plant material is direct gene transfer using co-transformation (Schocher RJ et al, (1986), Bio/Technology, 4: 1093-1096).

Co-transformation is a method that is based on the simultaneous taking up and integration of various DNA molecules (non-selectable and selectable genes) into the plant genome and that therefore allows the detection of cells that have been transformed with non-selectable genes.

Further means for inserting genetic material contained in a vector directly into a plant cell comprise using purely physical procedures, for example by microinjection using finely drawn micropipettes Neuhaus et al., (1987) Theor. Appl. Genet. 75:30-36 or by bombarding the cells with microprojectiles that are coated with the transforming DNA "Microprojectile Bombardment" (Wang Y-C et al, (1988), Plant Mol. Biol. 11: 433-439) or are accelerated through a DNA containing solution in the direction of the cells to be transformed by a pressure impact thereby being finely atomized into a fog with the solution as a result of the pressure impact EP-A-434,616!.

Microprojectile bombardment has been advanced as an effective transformation technique for cells, including cells of plants. In Sanford et al, (1987), Particulate Science and Technology 5:27-37 it was reported that microprojectile bombardment was effective to deliver nucleic acid into the cytoplasm of plant cells of Allium cepa (onion). Christou et al, (1988), Plant Physiol 87: 671-674 reported the stable transformation of soybean callus with a kanamycin resistance gene via microprojectile bombardment. Christou et al reported penetration at approximately 0.1 % to 5 % of cells. Christou further reported observable levels of NPTII enzyme activity and resistance in the transformed calli of up to 400 mg/l of kanamycin. McCabe et al, (1988), Bio/Technology 6: 923-926 report the stable transformation of Glycine max (soybean) using microprojectile bombardment. McCabe et al further report the recovery of a transformed R₁ plant from an Rₒ chimaeric plant.

The transformation of maize plants, including elite maize plants, by microprojectile bombardment can be carried out according to the general protocol described for example in EP-A 478 502.

The list of possible transformation methods given above by way of example is not claimed to be complete and is not intended to limit the subject of the invention in any way.

It is further recognized that the components of the expression cassette may be modified to increase expression. For example, truncated sequences, nucleotide substitutions or other modifications may be employed.

It may also be beneficial to remove nucleotides in the T7 promoter sequence to prevent potential stem-loop structures in the RNA. Such nucleotides can be removed, for example, using PCR technology as set forth below in the Experimental Section. Likewise, a poly A chain may be included in the expression cassette adjacent to the terminator. For example, in expression cassette 2, about 25 to about 90 A nucleotides may be inserted 5' to the T7 terminator.

Other methods such as transplicing may also be employed utilizing the splice donor and splice acceptor sites of known genes such as the Adhl gene of maize. See. Dennis et al., (1984), Nucleic Acids Research, 12:3983-4000. Such a system involves three expression cassettes. The following are typical cassettes which could be utilized in a T7 system. Cassette 1 comprises an anther-specific promoter operably linked to a nucleotide sequence encoding a transactivator, e.g. T7 polymerase.

Cassette 2 comprises the target nucleotide sequence, T7 promoter, operably linked to a nucleotide sequence comprising a splice acceptor site. The acceptor site is operably linked to a nucleotide sequence comprising the 3' portion of the coding region of a polypeptide or RNA capable of disrupting the formation of viable pollen.

Cassette 3 comprises a nucleotide sequence encoding a promoter capable of directing expression in anther tissue, operably linked to a nucleotide sequence comprising the 5' coding region of the polypeptide or RNA capable of disrupting the formation of viable pollen which is operably linked to a splice donor site. As discussed earlier, the cassettes may also comprise leader sequences, terminators, etc. Parent plant 1 can be stably transformed with cassette 1, or alternatively, cassettes 1 and 3 while Parent plant 2 will contain cassettes 2 and 3, or alternatively cassette 2, respectively. In either situation crossing Parent 1 and Parent 2 results in male-sterile progeny.

Transformed plants are regenerated. The presence of the stably integrated expression cassette into the transformed parent plants may be ascertained by southern hybridization techniques or PCR analysis, known in the art. Expression of the transactivator may be determined by utilizing northern blot techniques.

Therefore, the present system can be utilized in any plant which can be transformed and regenerated. This includes preferably plants of the dicotyledonous and monocotyledonous class. Especially preferred within the scope of the invention is the use of monocotyledonous plants of the Graminaceae family involving the following plants: Lolium, Zea, Triticum, Triticale, Sorghum, Saccharum, Bromus, Oryzae, Avena, Hordeum, Secale and Setaria.

Accordingly, this invention relates preferably also to the use of plants from the *Graminaceae* family, including their sexual and asexual progeny, that can be regenerated from plant material selected from the group consisting of protoplasts, cells, calli, tissues, organs, seeds, embryos, pollen, ovules, zygotes, etc. for the production of male-sterile transgenic plants.

The method eliminates the necessity of manipulating floral structures and avoids the necessity of hand emasculation and fertilization.

The parent plants containing the respective stably integrated expression cassettes are both male fertile and can be made homozygous and maintained indefinitely. To obtain male-sterile seed, homozygous lines of parent 1 and 2 are crossed using a technique such as detasseling of one line and using the other as a pollinator, such that no self seed is produced. The male-sterile offspring can then be utilized as female parents in any cross to produce hybrid seed. About 75% of the resulting hybrid seeds will give rise to male fertile plants. Thus, for the purpose of producing hybrid seed, standard crossing of different lines with the male-sterile plants and subsequent analysis of the progeny to select a line with superior agronomic traits are performed. See, generally, International Patent Application Number WO 90/08828.

While the T7 polymerase system is useful in the above-described dual system for the production of male-sterile plants, it is also recognized that a T7 expression system can be utilized for high level expression of nucleotide sequences in plants. The system also provides tissue-specific expression or other selective expression of coding sequences in a plant.

In this manner, a single plant can be transformed with two expression cassettes. A first expression cassette comprises a T7 polymerase operably linked to a promoter capable of directing expression in a plant cell. Any promoter capable of directing expression can be utilized and can be chosen for specific expression; e.g. tissue-specific promoter, developmental stage-specific promoter, inducible promoter, etc. Specific promoters, for example, include chemical inducible promoters (EP-A 0 332 104) and seed specific promoters (Ellis et al., (1988), Plant Mol. Biol., 10:203-214).

Especially suitable are expression signals originating from genes of plants or plant viruses. Examples of suitable promoters and terminators are those of the Cauliflower Mosaic Virus genes (CaMV) or homologous DNA sequences that still have the chacteristics properties of the mentioned expression signals. Also suitable are bacterial expression signals, especially the expression signals of the nopaline synthase genes (nos) or the opine synthase genes (ocs) from the Ti-plasmids of Agrobacterium tumefaciens. Also to be mentioned here are, for example, ubiquitine promoters, actin promoters, histone promoters and tubulin promoters. Other suitable promoters are an amylase promoter (a-amylase promoter) and an ABA (abscisic acid) inducible promoter.

The expression signals may also comprise tissue-preferential or tissue-specific promoters. The term tissue-preferential promoter is used to indicate that a given expression signal will promote a higher level of transcription of an associated expressible DNA, or of expression of the product of the said DNA as indicated by any conventional RNA or protein assay, or that a given DNA sequence will demonstrate some differential effect; i.e., that the transcription of the associated DNA sequences or the expression of a gene product is greater in some tissue than in all other tissues of the plant. For example, the tissue-preferential promoter may direct higher expression of an associated gene product in leaves, stems, roots and/or pollen than in seed. One example of a tissue-preferential promoter, which may be suitably used within the scope of the present invention, is a pith-preferred promoter isolated from a maize TrpA gene as disclosed in WO 93/07278.

The term tissue-specific promoter is used to indicate that a given regulatory DNA sequence will promote transcription of an associated expressible DNA sequence entirely in one or more tissues of a plant, or in one type of tissue, while essentially no transcription of that associated coding DNA sequence will occur in all other tissues or types of tissues of the plant. Numerous promoters whose expression are known to vary in a tissue specific manner are known in the art. One such example is the maize phosphoenol pyruvate carboxylase (PEPC), which is green tissue-specific. Other green tissue-specific promoters include chlorophyll a/b binding protein promoters and RubisCo small subunit promoters. Further to be mentioned here are, for example, pollen-specific promoters such as those obtainable from a plant calcium-dependent phosphate kinase (CDPK) gene.

A developmentally regulated promoter can also be used. Of course, in the present invention, any promoter which is functional in the desired host plant can be used to direct the expression of an associated gene.

It is often advantageous to incorporate a leader sequence between the promoter sequence and the adjacent coding DNA sequence, the length of the leader sequence being so selected that the distance between the promoter and the DNA sequence according to the invention is the optimum distance for expression of the associated structural gene. Suitable leader sequences include leader sequences of various lengths isolated from the 35S CaMV gene (Pierce et al, Plant Gene Systems and their Biology, (Alan R. Liss, Inc.) pp. 301-310). The preferred leader sequences are those isolated from the 35S CaMV gene, having a length from about 50 to about 130 nucleotides. The identification of other leader sequences is known in the art. See Della-Cioppa et al, (1987), Plant Physiology 84: 965-968

Further regulatory DNA sequence that may be used for the construction of chimaeric genes include, for example, sequences that are capable of regulating the transcription of an associated DNA sequence in plant tissues in the sense of induction or repression.

There are, for example, certain plant genes that are known to be induced by various internal and external factors, such as plant hormones, heat shock, chemicals, pathogens, oxygen deficiency, light, stress, etc.

Another class of genes that are suitable in plants comprises the light-regulated genes, especially the nuclear-coded gene of the small subunit of ribulose-1,5-biphosphate carboxylase (RUBISCO). Morelli et al, (1985), Nature 315: 200-204 have shown that the 5'-flanking sequence of a RUBISCO gene from the pea is capable of transferring light-inducibility to a reporter gene, provided the latter is linked in chimaeric form to that sequence. It has also been possible to extend this observation to other light-induced genes, for example the chlorophyll-a/b-binding protein.

A further group of regulatable DNA sequences comprises chemically regulatable sequences that are present, for example, in the PR (pathogenesis-related) protein genes of tobacco and are inducible by means of chemical regulators such as those described in EP-A-332,104.

The regulatable DNA sequences mentioned by way of example above may be of both natural and synthetic origin, or they may comprise a mixture of natural and synthetic DNA sequences.

The recombinant DNA sequences of the present invention may further comprise a signal sequence, which is operatively linked to the coding DNA sequence. The signal sequence is responsible for specialized transport of the associated peptide within the plant cell.

The signal sequence of the present invention may be any DNA sequence which is able to direct the transport of an associated polypeptide into one or more of the cellular compartments. The signal sequence is preferably a sequence which is translated into a signal peptide, which becomes separated from the peptide after transit of the peptide is complete. Signal sequences are useful for directing the polypeptide product of the coding DNA sequence to a desired location within the cell, such as to the mitochondria or to the endoplasmic reticulum, or to direct extracellular transport outside of the cell.

To be mentioned here are, for example, N-terminal signal peptides, which are involved in intracellular transport and which can be found at the N-terminal end of proteins transported via the endomembrane system. These signal sequences ensure that the said proteins first pass into the endoplasmic reticulum, where the signal peptide is split off proteolytically from the precursor protein as soon as it has fulfilled its function. By virtue of its specific function, this type of signal peptide sequence has been conserved to a high degree during evolution in all living cells, irrespective of whether they are bacteria, yeasts, fungi, animals or plants.

At the C-terminal end of vacuolar proteins, on the other side, sequences may be found that are involved in directing the expression of the associated coding part of the plant vacuole. Examples of these so-called 'vacuolar targeting' sequences are provided, for example, in EP-A 462,065.

Moreover, the DNA molecule may comprise further sections of sequence that code for peptide fragments which as a whole contribute towards improving the competence for admission into the vacuole, for example the propeptide fragment discovered by Matsuoka K. and Nakamura K. in the N-terminal extension of sporamine Matsuoka K. and Nakamura K., (1991), Proc Natl Acad Sci, USA, 88: 834-838.

Further signal sequences useful for the present invention are, for example, the signal sequence from the pathogenesis-related gene (PR-1) of tobacco, which is described in Cornellison et al, (1986), EMBO 5: 37-40; the yeast mitochondrial presequence; Schmitz et al, (1989) Plant Cell, 1: 783-791; the signal sequence from plant mitochondrial Rieske iron-sulfur protein, Huang et al, (1991), Proc Nat Acad Sci USA, 88: 10716-10720; mitochondrial and chloroplast targeting peptides, von Heijne et al, (1989), Eur J Biochem, 180: 535-545.

As described earlier, the first expression cassette may additionally comprise nuclear location signals, terminator sequences, plant translational consensus sequences, etc.

The second expression cassette comprises a coding sequence operably linked to a nucleotide sequence encoding T7 promoter. The second expression cassette may also comprise 5' leader sequences, terminator sequences, etc.

When both expression cassettes have been stably integrated into a single plant, the T7 polymerase will drive expression of the coding sequence operably linked to the T7 promoter.

It is recognized that the two expression cassettes may be part of a single vector or nucleic acid sequence or may be housed on separate vectors. Likewise, while a single plant in most instances will be transformed with each cassette, it may be beneficial at times to transform one plant, parent 1, with expression cassette 1 and another plant, parent 2, with expression cassette 2 and obtain progeny with both expression cassettes by crossing parents 1 and 2.

Because transcription by T7 RNA polymerase is highly active, this system may be utilized to increase the production of specific gene products which are produced in low quantities in plants. The method is also useful for increasing tissue or other specific gene products. Generally, at least about a two fold to greater than a 100 fold, more specifically about 4 fold to about 50 fold, increase in expression can be seen using a T7 system.

The T7 RNA polymerase is very selective for specific promoters that are rarely encountered in DNA unrelated to T7 DNA. Efficient termination signals are also rare. Therefore, the T7 RNA polymerase expression system can make complete transcripts of almost any DNA that is placed under control of a T7 promoter. Accordingly, the T7 expression system can be used to express a wide variety of products such as seed storage proteins with preferred amino acid composition; pharmaceutical proteins; proteins involved in starch, lipid or protein synthesis; insecticidal or disease resistance proteins; proteins which increase the nutritional quality of plants or seeds; antifungal, antibacterial or antiviral proteins; proteins that lead to the production of other proteins that render the plant resistant to insects or diseases; assembly proteins or proteins that are required for the production of other proteins; and the like.

Especially suitable for use in the T7 RNA polymerase expression system according to the invention are all those structural genes which upon expression lead to a protective effect in the transformed plant cells, also in the tissues developing therefrom and especially in the regenerated plants, for example increased resistance to pathogens (for example to phytopathogenic fungi, bacteria, viruses, etc.); resistance to chemicals [for example to herbicides (e.g. triazines, sulfonylureas, imidazolinones, triazole pyrimidines, bialaphos, glyphosate, etc.), insecticides or other biocides]; resistance to adverse environmental factors (for example to heat, cold, wind, adverse soil conditions, moisture, dryness, *etc.*).

Within the scope of this invention, special mention is to be made of structural genes that are associated with the control of plant pathogens and parasites.

Resistance to insects can be conferred, for example, by a gene coding for a polypeptide that is toxic to insects and/or their larvae, for example the crystalline protein of *Bacillus thuringiensis* [*B*.*t*.]. Especially suitable are synthetic *B*.*t*. genes such as those disclosed in, for example, Koziel MG *et al*, (1993), Bio/Technology 11: 194-200.

A second class of proteins mediating resistance to insects comprises the protease inhibitors. Protease inhibitors are a normal constituent of plant storage structures and are therefore normally located in vacuoles or protein bodies. It has been demonstrated that a Bowman-Birk protease inhibitor isolated from soybeans and purified inhibits the intestinal protease of *Tenebrio* larvae. The gene that codes for the trypsin inhibitor from the cowpea is described in Hilder *et al*, (1987), Nature 330: 160-163.

The majority of insects, for example, have a cuticular skeleton in which chitin micelles in lamellar layers are embedded in a base substance. A great many phytopathogenic fungi also contain chitin as an integral part of their hypha and spore structures, for example Basidiomycetes (smut and rust fungi), Ascomycetes and Fungi imperfecti (including Alternaria and Bipolaris, Exerophilum turcicum, Colletotricum, Gleocercospora and Cercospora). Chitinase is capable of inhibiting the mycelial growth of certain pathogens both *in vitro* and *in vivo*. A plant organ or tissue that is capable of expressing chitinase constitutively or in response to the penetration of a pathogen *can* therefore protect itself from attack by a large number of different fungi.

A further gene, which encodes an enzyme which presumably plays a central role in the plant's defence mechanism against pathogens is the β-1,3-glucanase gene, that may thus also be used for protecting plants against a fungal attack, alone or in combination with a chitinase gene.

A further class of genes that may be used within the T7 RNA polymerase expression system are those coding for the so-called lytic peptides. These are natural or synthetic peptides having anti-pathogenic activity which are capable of penetrating, lysing or otherwise damaging the cell membrane of pathogens. Representatives of such lytic peptides that may be used within the scope of the present invention are known both from animal sources [including insects] and from plant and microbial sources and include, for example, the defensins, cecropins, thionins and mellitins of mammals, and the defensins, magainins, attacins, dipterins, sapecins, caerulins and xenopsins of insects, and hybrids thereof. The amino acid sequences of various lytic peptides are shown in the following publications: WO 89/11291; WO 86/04356; WO 88/05826; US 4,810,777; WO 89/04371.

Lytic peptides in the broadest sense of the term are also to be understood as being compounds whose ability to penetrate, lyse or damage cell membranes is based on enzymatic activity, for example lysozymes and phospholipases.

Moreover, reciprocal use of expression and exogenous application may also be envisaged, the lytic peptides especially being suitable for the latter purpose, in conjunction with the auxiliaries and/or additives customarily used for this purpose.

Another class of genes that may be used in the scope of the present invention are thos coding for phospholipid transfer proteins disclosed, for example, in WO 92/20801.

A further class of genes that may be used within the scope of the present invention comprises genes which encode pathogenesis-related proteins [PRPs] such as PR-1A, PR-1B, PR-1C, PR-R major, PR-R minor, PR-P, PR-Q, PR-2, PR-2', PR-2'', PR-N, PR-O, PR-O', PR-4, SAR8.2a-e, cucumber chitinase/lysozyme, cucumber basic peroxidase, tobacco basic glucanase and tobacco basic chitinase/lysozyme, tobacco acidic chitinase/lysozyme. Examples of the above genes and proteins including chimeric genetic constructs comprising the said genes are provided in EP-A 392,225.

The DNA sequence according to the invention can also be used for the production of desirable and useful compounds in the plant cell as such or as part of a unit of higher organisation, for example a tissue, callus, organ, embryo or a whole plant.

Genes that may also be used within the T7 RNA polymerase expression system according to the invention include, for example, those which lead to increased or decreased formation of reserve or stored substances in leaves, seeds, tubers, roots, stems, etc. or in the protein bodies of seeds. The desirable substances that can be produced by transgenic plants include, for example, proteins, carbohydrates, amino acids, vitamins, alkaloids, flavins, perfumes, colourings, fats, etc..

There may also be associated with the DNA sequence according to the invention structural genes that code for pharmaceutically acceptable active substances, for example hormones, immunomodulators and other physiologically active substances.

The genes that can come into consideration within the scope of this invention therefore include, but are not limited to, for example, plant-specific genes, such as the zein gene from maize, the avenin gene from oats, the glutelin gene from rice, etc., mammal-specific genes, such as the insulin gene, the somatostatin gene, the interleukin genes, the t-PA gene, etc., or genes of microbial origin, such as the NPT II gene, etc. and synthetic genes, such as the insulin gene, etc..

Apart from naturally occurring structural genes that code for a useful and desirable property, within the scope of this invention it is also possible to use genes that have been modified previously in a specific manner using chemical or genetic engineering methods.

Furthermore, the broad concept of the present invention also includes genes that are produced entirely or patially by chemical synthesis. Genes or DNA sequences that may be used within the scope of the present invention are therefore both homologous and heterologous gene(s) or DNA and also synthetic gene(s) or DNA according to the definition given within the scope of the present invention. The insulin gene may be mentioned at this point as an example of a synthetic gene.

As discussed earlier, methods for manipulation of nucleic acid sequences and for transformation and regeneration of plants are known in the art.

Having generally described the invention, the following examples are offered by way of illustration and not by way of limitation.

### NON-LIMITING EXAMPLES

### General recombinant DNA techniques

Since many of the recombinant DNA techniques employed in this invention are a matter of routine for the person skilled in the art, it is better to give a short description of these generally used techniques here rather than to describe them every time they occur. Except where there is a specific indication to the contrary, all these procedures are described in the Maniatis *et al* (1982) reference.

### A. Cleaving with restriction endonucleases

A reaction batch typically contains about 50 to 500 µg/ml of DNA in the buffer solution recommended by the manufacturer, New England Biolabs, Beverly, MA.. 2 to 5 units of restriction endonucleases are added for each µg of DNA and the reaction batch is incubated for from one to three hours at the temperature recommended by the manufacturer. The reaction is terminated by heating at 65°C for 10 minutes or by extraction with phenol, followed by precipitation of the DNA with ethanol. This technique is also described on pages 104 to 106 of the Maniatis *et al* (1982) reference.

### B. Treatment of DNA with polymerase in order to produce blunt ends

50 to 500 µg/ml of DNA fragments are added to a reaction batch in the buffer recommended by the manufacturer, New England Biolabs. The reaction batch contains all four deoxynucleotide triphosphates in concentrations of 0.2 mM. The reaction takes place over a period of 30 minutes at 15°C and is then terminated by heating at 65°C for 10 minutes. For fragments obtained by cleaving with restriction endonucleases that produce 5'-projecting ends, such as EcoRI and BamHI, the large fragment, or Klenow fragment, of DNA polymerase is used. For fragments obtained by means of endonucleases that produce 3'-projecting ends, such as PstI and SacI, the T4 DNA polymerase is used. The use of these two enzymes is described on pages 113 to 121 of the Maniatis *et al* (1982) reference.

### C. Agarose gel electrophoresis and purification of DNA fragments from gels

Agarose gel electrophoresis is carried out in a horizontal apparatus, as described on pages 150 to 163 of the Maniatis *et al* reference. The buffer used is the tris-borate buffer described therein. The DNA fragments are stained using 0.5 µg/ml of ethidium bromide which is either present in the gel or tank buffer during electrophoresis or is added after electrophoresis. The DNA is made visible by illumination with long-wave ultraviolet light. If the fragments are to be separated from the gel, an agarose is used that gels at low temperature and is obtainable from Sigma Chemical, St. Louis, Missouri. After the electrophoresis, the desired fragment is cut out, placed in a plastics test tube, heated at 65°C for about 15 minutes, extracted three times with phenol and precipitated twice with ethanol. This procedure is slightly different from that described by Maniatis *et al* (1982) on page 170.

As an alternative, the DNA can be isolated from the agarose with the aid of the Geneclean kit (Bio 101 Inc., La Jolla, CA, USA).

### D. Addition of synthetic linker fragments to DNA ends

If it is desired to add a new endonuclease cleavage site to the end of a DNA molecule, the molecule is optionally first treated with DNA-polymerase in order to produce blunt ends, as described in the section above. About 0.1 to 1.0 µg of this fragment is added to about 10 ng of phosphorylated linker DNA, obtained from New England Biolabs, in a volume of 20 to 30 µl with 2 µl of T4 DNA ligase from New England Biolabs, and 1 mM ATP in the buffer recommended by the manufacturer. After incubation overnight at 15°C, the reaction is terminated by heating at 65°C for 10 minutes.
The reaction batch is diluted to about 100 µl in a buffer appropriate for the restriction endonuclease that cleaves the synthetic linker sequence. About 50 to 200 units of this endonuclease are added. The mixture is incubated for 2 to 6 hours at the appropriate temperature, then the fragment is subjected to agarose gel electrophoresis and purified as described above. The resulting fragment will then have ends with endings that were produced by cleaving with the restriction endonuclease. These ends are usually cohesive, so that the resulting fragment can then readily be linked to other fragments having the same cohesive ends.

### E. Removal of 5'-terminal phosphates from DNA fragments

During the plasmid cloning steps, treatment of the vector plasmid with phosphatase reduces the recircularisation of the vector (discussed on page 13 of the Maniatis *et al* reference). After cleavage of the DNA with the correct restriction endonuclease, one unit of calf intestinal alkaline phosphatase obtained from Boehringer-Mannheim, Mannheim, is added. The DNA is incubated at 37°C for one hour and then extracted twice with phenol and precipitated with ethanol.

### F. Linking of DNA fragments

If fragments having complementary cohesive ends are to be linked to one another, about 100 ng of each fragment are incubated in a reaction mixture of 20 to 40 µl containing about 0.2 unit of T4 DNA ligase from New England Biolabs in the buffer recommended by the manufacturer. Incubation is carried out for 1 to 20 hours at 15°C. If DNA fragments having blunt ends are to be linked, they are incubated as above except that the amount of T4 DNA ligase is increased to 2 to 4 units.

### G. Transformation of DNA into E. coli

*E. coli* strain HB101 is used for most of the experiments. DNA is introduced into *E. coli* using the calcium chloride method, as described by Maniatis *et al* (1982), pages 250 and 251.

### H. Screening of E. coli for plasmids

After transformation, the resulting colonies of *E. coli* are tested for the presence of the desired plasmid by means of a rapid plasmid isolation process. Two customary processes are described on pages 366 to 369 of the Maniatis *et al* (1982) reference.

### I. Large-scale isolation of plasmid DNA

Processes for the isolation of plasmids from *E. coli* on a large scale are described on pages 88 to 94 of the Maniatis *et al* (1982) reference.

### J. Cloning in M13 phage vectors

In the following description it is to be understood that the double-stranded replicative form of the phage M13 derivatives is used for routine processes, such as cleaving with restriction endonuclease, linking etc..
Unless there is a specific indication to the contrary, enzymes can be obtained from Boehringer, Biolabs (BRL). They are used in accordance with the manufacturer's instructions unless otherwise indicated.

### K. Southern blot analysis

The extracted DNA is first treated with restriction enzymes, then subjected to electrophoresis in a 0.8 % to 1 % agarose gel, transferred to a nitrocellulose membrane [Southern EM (1975)] and hybridised with the DNA to be detected which has previously been subjected to nick-translation (DNA-specific activities of 5 x 10⁸ to 10 x 10⁸ c.p.m./µg). The filters are washed three times for 1 hour each time with an aqueous solution of 0.03M sodium citrate and 0.3M sodium chloride at 65°C. The hybridised DNA is made visible by blackening an X-ray film over a period of 24 to 48 hours.

### EXPERIMENTAL

### Example 1: Addition of a plant translational consensus sequence to the T7 RNA polymerase gene

The translational start site of the T7 RNA polymerase gene [with the SV40 nuclear localization signal (NLS)] of pAR3283 (Dunn et al., Gene 68: 259-255 (1988) was modified to include a plant translational consensus sequence (Joshi, C.P., NAR 15, 6643-6653 (1987)). The BgIII to NruI fragment of pAR3283 was replaced with a BgIII - NruI PCR generated fragment in which the sequence TAAACAATG, following the BglII site, replaced the sequence before the T7 translational start site. The nucleotides after the translational start site were not modified to conform to the plant consensus sequence (TAAACAATGGCT) because an asparagine to alanine substitution would result.

### Example 2: Fusion of the 35S CaMV promoter to the T7 RNA polymerase gene

The T7 RNA polymerase gene containing the SV40 nuclear localization signal (NLS) and a plant translational consensus sequence was excised as a BglII - BamHI fragment and cloned into the BamHI site of pCIB710 (Rothstein et al., Gene 53:153-161 (1987)). The resulting plasmid, pJS175, contains the 35S CaMV promoter, T7 RNA polymerase gene (SV40 NLS, plant translational consensus sequence) and the 35S CaMV poly A addition site.

### Example 3: T7 promoter/terminator constructions and fusions to the GUS gene

The T7 promoter and T7 terminator from pET-3 (Rosenberg et al., Gene 56, 125 (1987)) was inserted as a BglII fragment into BamHI-cleaved pUC19 [New England Biolabs, Beverly, Maryland, USA] to make pAT26 and into BamHI-cleaved bluescript SK [Stratagene Cloning Systems, La Jolla, California, USA] to make pAT10. The 3' Sac I site of the GUS gene from pBI121 (Clontech) was adaptored to a Bam HI site and cloned into the Bam HI site between the T7 promoter and terminator of pAT10 to make pAT11. In pAT27, the nucleotides +9 to +26 of the T7 promoter were removed by PCR from pAT26 in order to eliminate a potential stem-loop structure in the RNA. A 35S CaMV poly A addition signal was inserted into the BamHI site of pAT27 by adding a BglII site by PCR on the 3' end of the fragment, resulting in pAT28. The GUS gene from pAT11 was inserted into the BamHI site of pAT28 to make pAT30 and into the BamHI site of pAT27 to make pAT32. pJS261 was constructed by replacing the T7 terminator of pAT26 with a BamHI - EcoRI fragment containing the 35S terminator, T7 terminator from pAT28. A BamHI fragment containing the TEV leader-GUS gene from pAT31 was then inserted in the BamHI site.

### Example 4: Translational constructs using the tobacco etch virus leader

The tobacco etch virus 5' nontranslated leader (nucleotides +6 to +143 of the genomic RNA, Allison et al., Virology 154:9-20 (1986)), with a BamHI site on the 5' end and a NcoI site on the 3' end, was translationally fused to a GUS gene (NcoI - SacI fragment) into bluescript SK to make pAT29. The SacI site of pAT29 was adaptored to BamHI and the BamHI fragment containing the TEV leader-GUS gene was inserted into the BamHI site of pAT28 to make pAT31.

### Example 5: Protoplast transformation and GUS fluorometric assays

Nicotiana tabacum protoplasts were transformed as described in Negrutiu, I. et al., PMB 8:363-373 (1987) and GUS fluorometric assays were performed as in Jefferson, R.A., PMB Reporter 5:387-405 (1987). Methods for production of maize protoplasts are described, for example, in example 6 and 43 of PCT application WO 93/07278, herein incorporated by reference.

Maize protoplast isolation - Protoplast Isolation Procedure:
1. The contents of 10 two day old maize 2717 Line 6 suspension cultures are pipetted into 50 ml sterile tubes and allowed to settle. All culture media is then removed and discarded.
2. Cells (3-5 ml Packed Cell Volume) are resuspended in 30 ml protoplast enzyme solution. Recipe follows:
3% Cellulase RS
1% Macerozyme R10 in KMC Buffer KMC Buffer (recipe for 1 liter)

| | |
|---|---|
| KCl | 8.65 g |
| MgCl2-6H2O | 16.47 g |
| CaCl2-2H2O | 12.50 g |
| MES | 5.0 g |

pH 5.6, filter sterilize
3. Mix cells well and aliquot into 100x25 mm petri dishes, about 15 ml per plate. Shake on a gyratory shaker for 4 hours to digest.
4. Pipette 10 ml KMC through each 100 micron sieve to be used. Filter contents of dishes through sieve. Wash sieve with an equal volume KMC.
5. Pipette sieved protoplasts carefully into 50 ml tubes and spin in a Beckman TJ-6 centrifuge for 10 minutes at 1000 rpm (500 x g).
6. Remove supernatant and resuspend pellet carefully in 10 ml KMC. Combine contents of 3 tubes into one and bring volume to 50 ml with KMC.
7. Spin and wash again by repeating the above step.
8. Resuspend all washed protoplasts in 50 ml KMC. Count in a hemocytometer. Spin protoplasts and resuspend at 8 x 106/ml in resuspending buffer (RS Buffer).

RS Buffer (recipe for 500 ml)
mannitol 27.33 g
CaCl2 (0.1 M stock) 75 ml
MES 0.5 g
pH 5.8, filter sterilize

### Example 6: Transcription from the T7 promoter in transient experiments

Maize protoplasts were cotransformed with the 35S promoter driving T7 RNA polymerase (pJS 175) and the T7 promoter/ GUS gene/ T7 terminator (pAT11). As a negative control, protoplasts were also cotransformed with a 35S promoter/ luciferase gene (pCIB1700) and with pAT11. Protoplasts transformed with 35S/ GUS (pCIB246) were a positive GUS control. Plasmid pCIB246 is constructed as follows.

The DdeI restriction site at nucleotide position 7482 of the CaMV genome [Franck et al., Cell, 21:285-294 (1980)] is modified by insertion of a 48 bp oligonucleotide containing several restriction enzyme sites including an NcoI (CCATGG) site, a SalI (GTCGAC) site, and an SstI (GAGCTC) site. This altered CaMV 35S promoter is inserted into a pUC19 vector that had been modified to destroy the vector's SstI and SalI sites. Thus, the CaMV 35S promoter of pCIB1500 contains unique SstI and SaII sites for cloning. pCIB1500 is digested with SstI/NcoI and ligated with the GUS gene obtained from pBI221 (Clontech Laboratories, Inc., Palo Alto, CA). The NcoI site is fused to the GUS gene such that the ATG of the NcoI site functions as the start codon for the translation of the GUS gene. The CaMV 35S polyadenylation and termination signals are used for the 3' end of the chimeric gene.

RNA was isolated from protoplasts according to the guanidinium thiocyantate-phenol-chloroform method described by Goodall et al., Methods in Enzymology 181:148-161 (1990). Duplicate northerns were probed with a T7 RNA polymerase and a GUS probe. Only RNA from the protoplasts transformed with pJS175 and pAT11 hybridized to the T7 RNA polymerase probe. RNA from protoplasts transformed with pJS175 alone and with pJS175/ pAT11 hybridized to the GUS probe, showing that T7 RNA polymerase is transcribing off the T7 promoter in plant cells. GUS RNA levels transcribed from the T7 promoter were 10-fold higher than the pCIB246 control.

### Example 7: GUS expression using the Tobacco Etch Virus leader for translation of T7 transcripts

Tobacco protoplasts were cotransformed with the 35S CaMV promoter driving T7 RNA polymerase (pJS175) and T7 promoter - GUS fusions with and without the TEV leader (pAT31, pJS261). GUS fluormotetric assays were done (Table I).
GUS enzyme activity (4-fold higher than the 35S/GUS control) was seen in T7 constructs only when the TEV leader was present.

**Table I.**

| Transient expression experiment using the TEV leader | |
|---|---|
| pCIB 246 | 35S/GUS |
| pJS 175 | 35S/T7 RNA polymerase |
| pJS 179 | 35S/luciferase |
| pAT11 | T7 promoter/ GUS/ T7 terminator |
| pJS261 | T7 promoter/ TEV leader/ GUS/ 35S terminator/ T7 terminator |
| pAT31 | T7 promoter (stem loop removed)/ TEV leader/ GUS/ 35S terminator/ T7 terminator |

| | Specific GUS Activity (nm MU/µg Protein/min.) | Fold increase over pCIB 246 |
|---|---|---|
| pCIB 246 | 20.5 ± 4 | |
| pJS 179/pAT11 | 0.021 ± 0.007 | |
| pJS 175/pAT11 | 0.013 ± 0.004 | |
| pJS 175/pJS261 | 14.17 ± 0.45 | 0.7 |
| pJS 175/pAT31 | 80.8 ± 5 | 3.9 |

### Example 8: Fusion of an anther-specific promoter to the T7 RNA polymerase gene

The T7 RNA polymerase gene containing the SV40 nuclear localization signal and a plant translational consensus sequence was excised as a Bgl II - Bam HI fragment and cloned into the Bam HI site of pLC250, the construction of which is described in example 17 of the European Patent Application Number 93810455.1 filed June 24, 1993. In pLC250, a tapetal-specific tobacco anther promoter, ant32, was cloned into the Sal I- Xba I sites of the Agrobacterium binary plasmid vector pBI101 (Clontech, Palo Alto, CA). The GUS gene had previously been removed from pBI101 with Sma I, Sac I and the Sac I site had been blunted. The resulting plasmid, pAT20, contains the ant32 tobacco anther promoter, the T7 RNA polymerase gene (SV40 NLS, plant translational consensus sequence) and a nos terminator.

The ant32 promoter can be obtained from the 2.0 kb 5' flanking region of the ant32 clone, the preparation of which is described in the European Patent Application Number 93810455.1 filed June 24, 1993. The DNA sequence of the ant32 genomic clone is provided in SEQ ID NO: 1.

### Example 9: Construction of plant transformation vectors

### Construction of pCIB710 and derivatives.

The construction of pCIB710 is described in example 4 of WO 93/07278. CaMV 35S Promoter Cassette Plasmids pCIB709 and pCIB710 are constructed as shown in Rothstein et al., Gene 53:153-161 (1987). pCIB710 contains CaMV promoter and transcription termination sequences for the 35S RNA transcript [Covey et al., Nucl. Acids. Res., 9:6735-6747 (1981)]. A 1149 bp BglII restriction fragment of CaMV DNA [bp 6494-7643 in Hohn et al., Current Topics in Microbiology and Immunology, 96: 194-220 and Appendices A to G (1982)] is isolated from from CaMV DNA by preparative agarose gel electrophoresis as described earlier The fragment is mixed with BamHI-cleaved plasmid pUC19 DNA, treated with T4 DNA ligase, and transformed into E. coli. (Note the BamHI restriction site in the resulting plasmid is destroyed by ligation of the BglII cohesive ends to the BamHI cohesive ends.)

The resulting plasmid, called pUC19/355, is then used in oligonucleotide-directed in-vitro mutagenesis to insert the BamHI recognition sequence GGATCC immediately following CaMV nucleotide 7483 in the Hohn reference. The resulting plasmid, pCIB710, contains the CaMV 35S promoter region and transcription termination region separated by a BamHI restriction site. DNA sequences inserted into this BamHI site will be expressed in plants by these CaMV transcription regulation sequences. (Also note that pCIB710 does not contain any ATG translation initiation codons between the start of transcription and the BamHI site).

pCIB710 is modified to produce pCIB709 by inserting a Bam HI fragment containing the coding sequenc for hygromycin phosphotransferase from pLG90 [Rothstein et al., Gene, 53:153-161 (1987)] in the Bam HI site.

pCIB709 is modified to produce pCIB996 by removing the ATG just upstream from the initiation codon of the hygromycin phosphotranserase gene using standard mutagenesis techniques while inserting a Bgl II restriction site at this location.

### Construction of plant transformation vectors containing an anther-specific promoter driving T7 RNA polymerase and the T7-promoter driving the Diphtheria toxin gene.

A plant transformation vector was constructed containing an anther-specific promoter driving T7 RNA polymerase and a T7 promoter driving the Diphtheria toxin A-chain (DTA) coding sequence (Palmiter et al, Cell 50:435-443). A T7 promoter/ TEV leader/DTA coding sequence/ 35S terminator/ T7 terminator cassette was made by excising the GUS gene from pAT30 with Bam HI and inserting in a TEV leader Bam HI - Nco I fragment from pAT29 and a DTA coding sequence Nco I - Bgl II fragment, resulting in pTG28. pTG32 is a vector for plant transformation containing both components and was made by adaptoring the 3' Eco RI site of pTG28 to Hind III and inserting the Hind III fragment into pAT20. The anther-specific promoter driving T7 RNA polymerase and the T7 promoter driving DTA can also be independently transformed into plants and then crossed in order to produce male-sterile plants. pTG35 contains only the T7 promoter driving DTA in a plant transformation vector and was constructed by adapting the 3' Eco RI site of pAT28 to Sal I and cloning into the Sal I site of the plant transformation vector pCIB905. For the construction of pCIB905, the 35S promoter, hygromycin resistance gene (Hy^{r}), and 35S 3' terminator were removed from pCIB996 as a KpnI, SalI fragment and inserted into the respective sites of pCIB200, the construction of which is described in example 14.1 of EP-A 0 462 065. Plants transformed with pTG35 can be crossed to pAT20 transformants.

### Example 10: Production of transgenic plants

Tobacco leaf discs were transformed with pTG32, pAT20 and pTG35 as described in Horsch et al., Science 227:1229-1231 (1985) and the presence of transforming DNA was confirmed using PCR.

### Example 11: Analysis of plants transformed with an anther-specific promoter driving T7 RNA polymerase and the T7 promoter driving DTA

The flower morphology of 13 plants transformed with pTG32 was observed. 11 plants were male-sterile and 9 of the 11 plants were shown to be female-fertile by backcrossing with wild-type tobacco.

### Example 12: Plant transformation vectors for GUS expression from the T7 promoter

The 35S CaMV promoter driving the T7 RNA polymerase and the T7 promoter driving the GUS gene were cloned into a plant transformation vector. As a control, the 35S CaMV promoter driving luciferase and the T7 promoter driving the GUS gene were also cloned. The T7 promoter (stem loop removed) /TEV leader/ GUS gene/ 35S terminator/T7 terminator were removed from pAT31 with Xba I, Eco RI and cloned into the plant transformation vector pCIB200 in pAT34. The 5' Sac I site of the 35S promoter/ T7 RNA polymerase/ nos terminator fragment was adaptored to Xba I (Sac I site destroyed) and cloned into the Xba I site pAT34 to make pAT35. For the control, a luciferase gene was cloned into the BamHI site of pCIB770 (Rothstein et al., Gene 53:153-161 (1987)) in pAT36. In pAT37, the EcoRI site of pAT31 was adapted to Sal I (Eco RI destroyed) and the Sal I fragment containing the T7 promoter/ TEV leader/ GUS gene/ 35S terminator/T7 terminator was cloned into the Sal I site of pAT36. In both pAT35 and pAT37, clones were chosen which have transcription of the two genes in opposite orientations away from each other.

### Example 13: Anti-sense inhibition using T7 polymerase and T7 promoters

In pCIB3217, the T7 promoter was inserted in an anti-sense direction after a 35S promoter/GUS/35S terminator cassette in pUC 19. This cassette is cloned into a plant transformation vector and is crossed to a plant transformed with 35S promoter/T7 RNA polymerase (pCIB3210). GUS enzyme activity of progeny carrying both T7 polymerase and the GUS gene/anti-sense T7 promoter is compared to progeny carrying only the GUS gene/anti-sense T7 promoter.

### Example 14: Construction of vectors containing the GAL4 binding site/ minimal 35S CAMV promoter fused to GUS and Diphtheria toxin

The GAL4 consensus binding site (Giniger et al., Cell 40:767-774 (1985) was fused to the CAMV 35S minimal promoter (-46 to +1, Benfey et al., EMBO 9: 1677-1684 (1990)) by incorporating the binding site into a PCR primer. The PCR primer used for addition of the GAL4 binding site upstream of the minimal -46 CaMV promoter was as follows: 5' GCGAAGCTTCGGAAGACTCTCCTCCGCTCGAGGCAAGACCCTTCCTCTCTATA-TA 3'. This 54 bp primer consists of a HindIII site, 17 bp GAL4 binding site, XhoI site and 23 bp of the CaMV promoter starting at position -46. The other PCR primer used in the reaction was as follows: 5' GCGATCTAGAATGGTCGACTAAGGGTTTCTT 3'. This 31 bp primer contains 21 bp of the 35S leader ending at +130 followed by a XbaI site. The addition of the GAL4 binding site to a minimal 35S promoter (-46 to + 130) was accomplished by performing a PCR reaction on pCIB246 (used as a template for the minmal 35S promoter).

The PCR generated band containing the binding site and the minimal promoter contained HindIII, XbaI ends and was cloned into plasmid vector pBI101. pLP3 contains the GAL4 binding site/ minimal 35S promoter/ GUS gene/ nos termininator in plant transformation vector pBI101 (with GUS removed). This cassette was excised from pLP3 with HindIII, EcoRI and cloned into bluescript to make pLP4. The GAL4 binding site/ minimal 35S promoter was fused to the DTA coding sequence. The GUS gene was first removed from pBI101 by excising with SmaI and SacI, blunting the SacI site, and religating the plasmid back together. The GAL4 binding site/ minimal 35S promoter was cloned into the HindIII, XbaI sites and the DTA gene was cloned as a BglII fragment into the BamHI site of the plasmid vector pBI101. pLP1 contains the GAL4 binding site/ 35S minimal promoter/ DTA coding sequence/ nos terminator in plant transformation vector pBI101 (with GUS removed).

### Example 15: GUS expression using the GAL4/VP16 transactivator

Maize protoplasts were cotransformed with a 35S promoter/GAL4/VP16 gene (pGAL4/VP1 - Goff et al., (1991). Genes and Development, 5:298-309) and a GAL4 binding site/ minimal 35S promoter/ GUS gene (pLP4). GUS fluorometric assays were performed (Table II). GUS enzyme activity was 20 fold higher from the GAL4 binding site/ minimal 35S promoter when the GAL4/VP16 transactivator was present.

**Table II.**

| Transient expression experiment using GAL4/VP16 Transactivation | |
|---|---|
| pLP4 | GAL4 binding site/ minimal 35S promoter/ GUS |
| pGAL4/VP1 | 35S promoter/Adh1 intron/ GAL4/ VP16 |

| | Specific GUS Activity (nm MU/µg protein) | Fold increase over pLP4 |
|---|---|---|
| no DNA | 0.20 ± 0.0 | |
| pLP4 | 0.26 ± 0.15 | |
| pLP4 / pGAL4/ VP1 | 5.25 ± 1.20 | 20 |

### Example 16: Fusion of an anther promoter to GAL4/VP16

The GAL4/ VP16 fusion was excised as a BamHI fragment from pGAL4/VP1 and inserted into the BamHI site of pLC250. The resulting plasmid, pLP2, contains an anther-specific promoter/ GAL4/VP16 / nos terminator in plant transformation vector pBI101 (with GUS removed). Transformants containing pLP2 can be crossed to plants transformed with pLP3 in order to get activation of the GUS gene or to plants transformed with pLP1 in order to produce male-sterile plants.

Although the foregoing invention has been described in some detail by way of illustration and example for purposes of clarity of understanding, it will be obvious that certain changes and modifications may be practiced within the scope of the appended claims.

## Claims

1. A method for producing male-sterile plants, said method comprising:
transforming a first parent plant cell with a first expression cassette, said cassette comprising a nucleotide sequence encoding an anther-specific 5' regulatory region operably linked to a nucleotide sequence which encodes a transactivator polypeptide;
regenerating a transformed plant, Parent 1, from said first transformed plant cell;
transforming a second parent plant cell with a second expression cassette comprising a target nucleotide sequence which is capable of being activated by said transactivator polypeptide operably linked to a nucleotide sequence which encodes anti-sense RNA or a polypeptide which disrupts the formation of viable pollen when expressed;
regenerating a transformed plant, Parent 2, from said second transformed plant cell; and
crossing said parent 1 with said parent 2 to obtain male-sterile offspring.

2. The method of claim 1, wherein said transactivator polypeptide is a T7 polymerase.

3. The method of claim 2, wherein said target nucleotide sequence is a T7 5' regulatory region.

4. The method of claim 2, wherein said expression cassette used to transform said parent 1 further comprises a nuclear localization signal sequence.

5. The method of claim 4, wherein said nuclear location signal is a viral nuclear localization signal from SV40.

6. The method of claim 1, wherein said transactivator polypeptide is a DNA binding protein.

7. The method of claim 6, wherein said DNA binding protein is GAL4/VP16 or GAL4/c1.

8. The method of claim 1, wherein said anti-sense RNA is capable of hybridizing to a message from a coding sequence that is critical to pollen formation or function.

9. The method of claim 1, wherein said polypeptide which disrupts the formation of viable pollen when expressed is selected from diphtheria toxin A, pectate lyase, T-urf13, gin recombinase, indole acetic acid-lysine synthetase, cytA toxin, APRT, RNAse, DNAse or protease.

10. The method of claim 9, wherein said polypeptide is diphtheria toxin A.

11. The method of claim 6, wherein said expression cassette of parent 2 further comprises a GAL4 binding site linked to a minimal 35S promoter operably linked to said nucleotide sequence which encodes anti-sense RNA or a polypeptide which will disrupt the formation of viable pollen.

12. The method of claim 2, wherein said expression cassette of parent 2 further comprises a T7 terminator sequence.

13. The method of claim 2, wherein said expression cassette of parent 2 further comprises a plant terminator sequence.

14. The method of claim 12, wherein said expression cassette of parent 2 further comprises a plant terminator sequence.

15. A method for producing hybrid seed from plants selected from those species of pollen producing plants which are capable of being genetically transformed, said method comprising:
(a) producing male-sterile plants by:
(i) transforming a first parent plant cell with a first expression cassette which comprises a nucleotide sequence encoding an anther-specific 5' regulatory region which is operably linked to a nucleotide sequence encoding a transactivator,
(ii) regenerating a transformed plant, Parent 1, from said first transformed plant cell;
(iii) transforming a second parent plant cell with an expression cassette which comprises a target nucleotide sequence which is activated by said transactivator operably linked to a nucleotide sequence which encodes anti-sense RNA or a polypeptide which will disrupt the formation of viable pollen;
(iv) regenerating a transformed plant, Parent 2, from said second transformed plant cell; and
(v) crossing said parent 1 with said parent 2 to produce male-sterile offspring; and
(b) crossing said male-sterile offspring with a selected fertile line to obtain hybrid seed.

16. A transgenic plant and the progeny thereof, which comprises a stably integrated expression cassette wherein said expression cassette comprises a nucleotide sequence encoding an anther-specific promoter which is operably linked to a nucleotide sequence encoding a transactivator capable of regulating a target nucleotide sequence.

17. A transgenic plant and the progeny thereof, which comprises a stably integrated expression cassette wherein said expression cassette comprises a target nucleic acid sequence, which is capable of being activated by a transactivator, operably linked to a nucleotide sequence which encodes anti-sense RNA or a polypeptide which will disrupt the formation of viable pollen.

18. A transgenic male-sterile plant and the progeny thereof, which comprises a stably integrated first expression cassette comprising a nucleotide sequence encoding an anther-specific promoter which is operably linked to a nucleotide sequence encoding a transactivator and a second expression cassette comprising a target nucleotide sequence, which is activated by said transactivator polypeptide, operably linked to a nucleotide sequence which encodes anti-sense RNA or a polypeptide which will disrupt the formation of viable pollen.

19. Hybrid seed produced from the method of claim 15 and comprising said expression cassettes.

20. A male-sterile plant produced by the method of claim 1 and comprising said expression cassettes.

21. A transgenic plant and the progeny thereof according to any one of claims 16 to 18, which is a maize plant.

22. A transgenic plant and the progeny thereof according to any one of claims 16 to 18, which is a wheat plant.

23. A transgenic plant and the progeny thereof according to any one of claims 16 to 18, which is a barley plant.

## Patentansprüche

1. Verfahren zur Herstellung von männlich-sterilen Pflanzen, wobei das Verfahren umfasst:
Transformieren einer ersten elterlichen Pflanzenzelle mit einer ersten Expressionskassette, wobei die Kassette eine Nucleotidsequenz umfasst, die eine Anthera-spezifische 5'-Regulatorregion kodiert, die mit einer Nucleotidsequenz operabel verknüpft ist, die ein Transaktivator-Polypeptid kodiert;
Regenerieren einer transformierten Pflanze, Elter 1, aus der ersten transformierten Pflanzenzelle;
Transformieren einer zweiten elterlichen Pflanzenzelle mit einer zweiten Expressionskassette, die eine Ziel-Nucleotidsequenz umfasst, die in der Lage ist, durch das Transaktivator-Polypeptid aktiviert zu werden, das mit einer Nucleotidsequenz operabel verknüpft ist, die Antisense-RNA oder ein Polypeptid kodiert, das die Bildung von überlebensfähigem Pollen bei Expression unterbricht;
Regenerieren einer transformierten Pflanze, Elter 2, aus der zweiten transformierten Pflanzenzelle; und
Kreuzen von Elter 1 mit Elter 2 unter Erhalt von männlich-sterilen Abkömmlingen.

2. Verfahren nach Anspruch 1, wobei das Transaktivator-Polypeptid eine T7-Polymerase ist.

3. Verfahren nach Anspruch 2, wobei die Ziel-Nucleotidsequenz eine T7-5'-Regulatorregion ist.

4. Verfahren nach Anspruch 2, wobei die Expressionskassette, die zur Transformation von Elter 1 verwendet wird, außerdem eine nukleäre Lokalisierungssignal-Sequenz umfasst.

5. Verfahren nach Anspruch 4, wobei das nukleäre Lokalisierungssignal ein virales nukleäres Lokalisierungssignal aus SV40 ist.

6. Verfahren nach Anspruch 1, wobei das Transaktivator-Polypeptid ein DNA-bindendes Protein ist.

7. Verfahren nach Anspruch 6, wobei das DNA-Bindungsprotein GAL4/VP16 oder GAL4/c1 ist.

8. Verfahren nach Anspruch 1, wobei die Antisense-RNA zur Hybridisierung an einer Botschaft aus einer kodierenden Sequenz in der Lage ist, die für die Pollenbildung oder -funktion kritisch ist.

9. Verfahren nach Anspruch 1, wobei das Polypeptid, das die Bildung von überlebensfähigem Pollen bei Expression unterbricht, ausgewählt ist aus Diphtherietoxin A, Pectatlyase, T-URF 13, Gin-Rekombinase, Indolessigsäure-Lysinsynthetase, CytA-Toxin, APRT, RNAse, DNAse oder Protease.

10. Verfahren nach Anspruch 9, wobei das Polypeptid ein Diphtherietoxin A ist.

11. Verfahren nach Anspruch 6, wobei die Expressionskassette von Elter 2 außerdem eine GAL-4-Bindungsstelle umfasst, die mit einem S35-Minimalpromotor verknüpft ist, der mit der Nucleotidsequenz operabel verknüpft ist, die Antisense-RNA oder ein Polypeptid kodiert, das die Bildung von überlebensfähigem Pollen unterbricht.

12. Verfahren nach Anspruch 2, wobei die Expressionskassette von Elter 2 außerdem eine T7-Terminatorsequenz umfasst.

13. Verfahren nach Anspruch 2, wobei die Expressionskassette von Elter 2 außerdem eine Pflanzen-Terminatorsequenz umfasst.

14. Verfahren nach Anspruch 12, wobei die Expressionskassette von Elter 2 außerdem eine Pflanzen-Terminatorsequenz umfasst.

15. Verfahren zur Herstellung von hybridem Saatgut aus Pflanzen, die aus denjenigen Spezies von Pollen-produzierenden Pflanzen ausgewählt sind, die in der Lage sind, genetisch transformiert zu werden, wobei das Verfahren umfasst:
a) Herstellen von männlich-sterilen Pflanzen durch
i) Transformieren einer ersten elterlichen Pflanzenzelle mit einer ersten Expressionskassette, die eine Nucleotidsequenz umfasst, die eine Anthera-spezifische 5'-Regulatorregion codiert, die mit einer Nucleotidsequenz, die einen Transaktivator kodiert, operabel verknüpft ist.
ii) Regenerieren einer transformierten Pflanze, Elter 1, aus der ersten transformierten Pflanzenzelle;
iii) Transformieren einer zweiten elterlichen Pflanzenzelle mit einer Expressionskassette, die eine Ziel-Nucleotidsequenz umfasst, die durch den Transaktivator aktiviert wird, der mit einer Nucleotidsequenz operabel verknüpft ist, die Antisense-RNA oder ein Polypeptid kodiert, das die Bildung von überlebensfähigem Pollen unterbricht;
iv) Regenerieren einer transformierten Pflanze, Elter 2, aus der zweiten transformierten Pflanzenzelle; und
v) Kreuzen von Elter 1 mit Elter 2 unter Erzeugung männlich-steriler Nachkommen; und
b) Kreuzen der männlich-sterilen Abkömmlinge mit einer ausgewählten fruchtbaren Linie unter Erhalt von hybridem Saatgut.

16. Transgene Pflanze und die Nachkommen davon, die eine stabil integrierte Expressionskassette umfasst, wobei die Expressionskassette eine Nucleotidsequenz umfasst, die einen Anthera-spezifischen Promotor kodiert, der mit einer Nucleotidsequenz operabel verknüpft ist, die einen Transaktivator kodiert, der in der Lage ist, eine Ziel-Nucleotidsequenz zu regulieren.

17. Transgene Pflanze und die Nachkommen davon, die eine stabil integrierte Expressionskassette umfasst, wobei die Expressionskassette eine Ziel-Nucleinsäuresequenz umfasst, die in der Lage ist, durch einen Transaktivator aktiviert zu werden, der mit einer Nucleotidsequenz operabel verknüpft ist, die Antisense-RNA oder ein Polypeptid kodiert, das die Bildung von überlebensfähigem Pollen unterbricht.

18. Transgene männlich-sterile Pflanze und die Nachkommen davon, die eine stabil integrierte erste Expressionskassette, die eine Nucleotidsequenz umfasst, die einen Anthera-spezifischen Promotor kodiert, der mit einer Nucleotidsequenz operabel verknüpft ist, die einen Transaktivator kodiert, und eine zweite Expressionskassette, die eine Ziel-Nucleotidsequenz umfasst, die durch das Transaktivator-Polypeptid aktiviert wird, das mit einer Nucleotidsequenz operabel verknüpft ist, die Antisense-RNA oder ein Polypeptid kodiert, das die Bildung von überlebensfähigem Pollen unterbricht, umfasst.

19. Hybrides Saatgut, das durch das Verfahren nach Anspruch 15 hergestellt wird und die Expressionskassetten umfasst.

20. Männlich-sterile Pflanze, die durch das Verfahren nach Anspruch 1 hergestellt wird und die Expressionskassetten umfasst.

21. Transgene Pflanze und die Nachkommen davon nach einem der Ansprüche 16 bis 18, die eine Maispflanze ist.

22. Transgene Pflanze und die Nachkommen davon nach einem der Ansprüche 16 bis 18, die eine Weizenpflanze ist.

23. Transgene Pflanze und die Nachkommen davon nach einem der Ansprüche 16 bis 18, die eine Gerstenpflanze ist.

## Revendications

1. Procédé pour produire des plantes à stérilité mâle, ledit procédé comprenant :
la transformation d'une première cellule végétale parentale avec une première cassette d'expression, ladite cassette comprenant une séquence nucléotidique codant une région régulatrice en 5' spécifique d'anthère liée de manière fonctionnelle à une séquence nucléotidique qui code un polypeptide transactivateur ;
la régénération d'une plante transformée, le parent 1, à partir de ladite première cellule végétale transformée ;
la transformation d'une seconde cellule végétale parentale avec une seconde cassette d'expression comprenant une séquence nucléotidique cible qui est capable d'être activée par ledit polypeptide transactivateur liée de manière fonctionnelle à une séquence nucléotidique qui code un ARN antisens ou un polypeptide qui interrompt la formation de pollen viable quand il est exprimé ;
la régénération d'une plante transformée, le parent 2, à partir de ladite seconde cellule végétale transformée ;
et
le croisement dudit parent 1 avec ledit parent 2 pour obtenir une descendance à stérilité mâle.

2. Procédé selon la revendication 1 où ledit polypeptide transactivateur est une polymérase de T7.

3. Procédé selon la revendication 2 où ladite séquence nucléotidique cible est une région régulatrice en 5' de T7.

4. Procédé selon la revendication 2 où ladite cassette d'expression utilisée pour transformer ledit parent 1 comprend en outre une séquence signal de localisation nucléaire.

5. Procédé selon la revendication 4 où ledit signal de localisation nucléaire est un signal de localisation nucléaire viral provenant de SV40.

6. Procédé selon la revendication 1 où ledit polypeptide transactivateur est une protéine liant l'ADN.

7. Procédé selon la revendication 6 où ladite protéine liant l'ADN est GAL4/VP16 ou GAL4/c1.

8. Procédé selon la revendication 1 où ledit ARN antisens est capable de s'hybrider à un message provenant d'une séquence codante qui est critique pour la formation ou la fonction du pollen.

9. Procédé selon la revendication 1 où ledit polypeptide qui interrompt la formation de pollen viable quand il est exprimé est choisi parmi la toxine diphtérique A, la pectate lyase, T-urf13, la gin recombinase, l'acide indole acétique-lysine synthétase, la toxine cytA, APRT, une RNase, une DNase ou une protéase.

10. Procédé selon la revendication 9 où ledit polypeptide est la toxine diphtérique A.

11. Procédé selon la revendication 6 où ladite cassette d'expression du parent 2 comprend en outre un site de liaison de GAL4 lié à un promoteur 35S minimal lié de manière fonctionnelle à ladite séquence nucléotidique qui code un ARN antisens ou un polypeptide qui interrompra la formation de pollen viable.

12. Procédé selon la revendication 2 où ladite cassette d'expression du parent 2 comprend en outre une séquence terminatrice de T7.

13. Procédé selon la revendication 2 où ladite cassette d'expression du parent 2 comprend en outre une séquence terminatrice végétale.

14. Procédé selon la revendication 12 où ladite cassette d'expression du parent 2 comprend en outre une séquence terminatrice végétale.

15. Procédé pour produire des graines hybrides à partir de plantes choisies parmi les espèces de plantes produisant du pollen qui sont capables d'être transformées génétiquement, ledit procédé comprenant :
(a) la production de plantes à stérilité mâle par :
(i) transformation d'une première cellule végétale parentale avec une première cassette d'expression qui comprend une séquence nucléotidique codant une région régulatrice en 5' spécifique d'anthère qui est liée de manière fonctionnelle à une séquence nucléotidique codant un transactivateur ;
(ii) régénération d'une plante transformée, le parent 1, à partir de ladite première cellule végétale transformée ;
(iii) transformation d'une seconde cellule végétale parentale avec une cassette d'expression qui comprend une séquence nucléotidique cible qui est activée par ledit transactivateur liée de manière fonctionnelle à une séquence nucléotidique qui code un ARN antisens ou un polypeptide qui va interrompre la formation de pollen viable ;
(iv) régénération d'une plante transformée, le parent 2, à partir de ladite seconde cellule végétale transformée ; et
(v) croisement dudit parent 1 avec ledit parent 2 pour produire une descendance à stérilité mâle ; et
(b) le croisement de ladite descendance à stérilité mâle avec une lignée fertile choisie, pour obtenir des graines hybrides.

16. Plante transgénique et ses descendants, comprenant une cassette d'expression intégrée de manière stable où ladite cassette d'expression comprend une séquence nucléotidique codant un promoteur spécifique d'anthère qui est liée de manière fonctionnelle à une séquence nucléotidique codant un transactivateur capable de réguler une séquence nucléotidique cible.

17. Plante transgénique et ses descendants, comprenant une cassette d'expression intégrée de manière stable où ladite cassette d'expression comprend une séquence d'acide nucléique cible, qui est capable d'être activée par un transactivateur, liée de manière fonctionnelle à une séquence nucléotidique qui code un ARN antisens ou un polypeptide qui va interrompre la formation de pollen viable.

18. Plante à stérilité mâle transgénique et ses descendants; comprenant une première cassette d'expression intégrée de manière stable comprenant une séquence nucléotidique codant un promoteur spécifique d'anthère qui est liée de manière fonctionnelle à une séquence nucléotidique codant un transactivateur et une seconde cassette d'expression comprenant une séquence nucléotidique cible, qui est activée par ledit polypeptide transactivateur, liée de manière fonctionnelle à une séquence nucléotidique qui code un ARN antisens ou un polypeptide qui va interrompre la formation de pollen viable.

19. Graines hybrides produites par le procédé de la revendication 15 et comprenant lesdites cassettes d'expression.

20. Plante à stérilité mâle produite par le procédé de la revendication 1 et comprenant lesdites cassettes d'expression.

21. Plante transgénique et ses descendants, selon l'une quelconque des revendications 16 à 18 qui est une plante de type maïs.

22. Plante transgénique et ses descendants, selon l'une quelconque des revendications 16 à 18 qui est une plante de type blé.

23. Plante transgénique et ses descendants, selon l'une quelconque des revendications 16 à 18 qui est une plante de type orge.


### SEQUENCE LISTING

(1) GENERAL INFORMATION:
   (i) APPLICANT:
      (A) NAME: CIBA-GEIGY AG
      (B) STREET: Klybeckstr. 141
      (C) CITY: Basel
      (E) COUNTRY: SCHWEIZ
      (F) POSTAL CODE (ZIP): 4002
      (G) TELEPHONE: +41 61 69 11 11
      (H) TELEFAX: + 41 61 696 79 76
      (I) TELEX: 962 991
   (ii) TITLE OF INVENTION: Methods for the production of hybrid seed
      Sequences and Recombinant DNA Sequences
   (iii) NUMBER OF SEQUENCES: 1
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patent In Release #1.0, Version #1.25 (EPO)
(2) INFORMATION FOR SEQ ID NO:1:
   (i) SEQUENCE CHARACTERISTICS:
      (A) LENGTH: 3706 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Nicotiana tabacum
      (C) INDIVIDUAL ISOLATE: Ant32 genomic clone
   (vii) IMMEDIATE SOURCE:
      (B) CLONE: pCIB950
   (ix) FEATURE:
      (A) NAME/KEY: TATA_signal
      (B) LOCATION: 1971..1975
   (ix) FEATURE:
      (A) NAME/KEY: CDS
      (B) LOCATION: 2076..3422
   (ix) FEATURE:
      (A) NAME/KEY: misc_feature
      (B) LOCATION: 2009
      (D) OTHER INFORMATION: /note= "Putative transcription start site"
   (xi) SEQUENCE DESCRIPTION: SEQ ID NO:1:
   CTGCAGTAAG GGGGATATTC AGAGACTCAA CTTAATCAAT ATTTGGCCCA AATTTGGCCT 60
   GCCGCGTCAC CCAAGGCATC GCATCAGTGT AATTCTCTTC GCAATCTGAT TTTTGCTCTG 120
   CTACCCTTCA TGAAAAAAGT CATAACTTCT TGTAGGAAAT ATTGGAATGA TAAATGGTTT 180
   GATGTTCTGG AAACTAGACT CACAGAAAAT TCATTTGATA TATAGCTAAT AGCTCAATTC 240
   GTAATGCATT CGGAGATATG ATTGTTTGAA GTTACATCAT ATGCGAGTAT GCTCGCTTTC 300
   TTCTCTTAAA CCTTTTCTAT TTGTTCCAAA CTACTTCTTC TCACTTATAG ATGTCCATAT 360
   AACTTTACAA ACATGAGATT TAGGTATTAC ACACCTTCAA AACTTTTCGA ACACACGTGT 420
   GTCTACTTAG GGCTTGAACC AGAACGTAAT ACTTAACGAT TTTCGGGGCA TTACATGCAT 480
   ACACCACTGT TAACAGGAAA ATTGCTTTCA TTAAATTATA ACATTGGATT TGGTGTGCAC 540
   TAAGTTCCTA TGCTTAATTG TTATGAACAT GAGTACTTTG CTTTCTCCCT TTGGTGGTGC 600
   ATACTTGTTT GTGGATATAT ATCGAGAATA ATAATGTGAG TGAATAGATA TTGTCTATTA 660
   TTTAACTTTA ATTTGCACCG CTACTTGTTC ACCACATTGG GATTCAATTG GGTGACTCGG 720
   CATATTTATC AATTAATATT CATCTAATGA GAACTCTTGC AAATTCTGTT ATAGGTTCTT 780
   AGTAGCATCA GCTGCATATC ATGTAAACTA AGAGTCAATA TGCTCACTTG TCAGTAAAAA 840
   AGAGTCATTA TCCTCACTTA TGTCATTTAC TCTATAGCTA TATTGGAGGC ATTATGTTAA 900
   TGGATTCCTA ATAATACCAA ATTACACCTT ATATGAGTCA TTGTTGGACA GAGTTTATCA 960
   ATACCTATAT ATTAGTGTAC TCTTATTCTT GCTCTTTGTG AGTATTAATA TGATGACTAT 1020
   ATTGACAGCA TTTGCATGAT GATGAGTGGG GCAGGAGACG CACAAAGTTT GTACCATAGA 1080
   GGAAGTTCGA GTTCTGTGAT AATCTTGGAA GAAAGTATAG TTATATTCTT TCTCCCCACC 1140
   TTGTTGATTT CCGACTTGTT TGAAGTTTGC TCCTTGTTGC TGTCACAATT GTATTCATGT 1200
   TAAGTTCTTT ATGAAGTTGG GTTGACGTTC AAATCTCATA CGCATGTTTG TTGCCTCTTT 1260
   TTATTTGTCT ATGGGGGTTG CATCAGTTGT CTCAGATCAA GATGGGAGCA TATTACTGCT 1320
   CCAAAGGTTT GGTTGTCCTT GGTAGTAACT AGTTCATGTG CAGGTTGGCT GCTCTGTTTG 1380
   ATTCTGCTTT GAGAACTTAA AGCTTTCATT TACTCAATTA TCAAATATCT GGGGTTTAAT 1440
   GGGCTCAAAT CACCCTTATA CAAACACCTT TTGTTTCCCT TATCAATGAA TGAACGAATT 1500
   TCCTTTGAGT TGTGAATGTA ATAAGGGTGT GAAAGAGGAG TTTTCGTTGT TAAATTGGCG 1560
   TTTGAAAGGT TCTCCCTTTT GTTCTTTTTT CGGCTTTTAC TTTTATATAC TGATAGTCTA 1620
   AGAAACTTTT TACACTATCA AGTTGCCTAA AAGATAGCTA CATGAGTAAC TTGTTACAAC 1680
   CGGTTAAATT ACACTAATAT TACAAATAAA AGTAAATCAG TAATATAAAA GTTATTTACA 1740
   TAGTCAATAT ATATAATTTA AATCCTTTTC TATTTTTTCT CGAGGGGTTT GGATTTTTAT 1800
   TTTAGTTGGC TCTTAAGACT TGTGCATGTA CATTCTTGAG AAAATAACTC TGTTCATGAG 1860
   AAAGCTACCT TAACTAACTA ACGTACTTCA CGGCCGAAAC AAAATCATAC AAATAACACA 1920
   TTTCTTTGTG GTTACCTTAA AATTTGGCCA TGAAACTTGG TCTGTTCGAT TATATCTTTA 1980
   AATACTACTA CCATCTACCA CACACTCTCC TCTGTCAAGA TAACAATAAA AGAATAAAAA 2040
   GATTAACCAA AAACGATATA CATATTTAGG ACAGA ATG AAG GTT AGC TTG AAG   2093
                                         Met Lys Val Ser Leu Lys
                                          1               5
   CAC CAC TGG GTA GTG AAG CCA GCA GAG GCA ACA TGG AAT GGC ACT GTC   2141
   His His Trp Val Val Lys Pro Ala Glu Ala Thr Trp Asn Gly Thr Val
               10                  15                  20
   TCC TTA TCG GAG TGT GAT CAA ACT TTT GCT GTA ACT CAT GTA CCA ACC   2189
   Ser Leu Ser Glu Cys Asp Gln Thr Phe Ala Val Thr His Val Pro Thr
   25 30 35
   ATT TAT TAC TAC AGG TTT TGC CAT GAT TGT CTT CCA TCA ACA GAC AAT 2237
   Ile Tyr Tyr Tyr Arg Phe Cys His Asp Cys Leu Pro Ser Thr Asp Asn
   40 45 50
   ATC ATC AAA ACC CTC AGG ACC TCA CTA AGC AAA GCA TTA GTA CAC TTC 2285
   Ile Ile Lys Thr Leu Arg Thr Ser Leu Ser Lys Ala Leu Val His Phe
   55 60 65 70
   TAT CCA TTG TCT GGT CGT TTG CGA TGG ATC GCT GGG TCC CGC CTC GAG 2333
   Tyr Pro Leu Ser Gly Arg Leu Arg Trp Ile Ala Gly Ser Arg Leu Glu
   75 80 85
   CTC GAC TGT AAT GCC TCG GGA ATC GTG CTC ATG GAA GCT GAA ACC GAA 2381
   Leu Asp Cys Asn Ala Ser Gly Ile Val Leu Met Glu Ala Glu Thr Glu
   90 95 100
   GCC AAA CTA GAT GAT CTT GGC GAT TTC TCG CCA TCC CCT GAC TTG AAC 2429
   Ala Lys Leu Asp Asp Leu Gly Asp Phe Ser Pro Ser Pro Asp Leu Asn
   105 110 115
   AGC TTG TTT CCC CGT GTA GAC TAC ACA ATC CCA ATT GAT GAA CTC CCT 2477
   Ser Leu Phe Pro Arg Val Asp Tyr Thr Ile Pro Ile Asp Glu Leu Pro
   120 125 130
   TTG TTG TTT GTT CAG CTT ACT AAG TTT CAG TGT GGT GGT ATT GCT CTG 2525
   Leu Leu Phe Val Gln Leu Thr Lys Phe Gln Cys Gly Gly Ile Ala Leu
   135 140 145 150
   AGT TTT GCA ATA TCA CAT GCT GTA GTT GAT GGC CAA AGT GCT CTT TAC 2573
   Ser Phe Ala Ile Ser His Ala Val Val Asp Gly Gln Ser Ala Leu Tyr
   155 160 165
   TTC CTC ACC GAA TGG GCT AGC CTT GCT CGC GGA GAG CCA TTA GGG AAC 2621
   Phe Leu Thr Glu Trp Ala Ser Leu Ala Arg Gly Glu Pro Leu Gly Asn
                   170                     175                     180
   GAA CCT TTT CAT GAT CGA AAA TTC CTC CGA GCA GGG GAA CCT CCA ATT    2669
   Glu Pro Phe His Asp Arg Lys Phe Leu Arg Ala Gly Glu Pro Pro Ile
           185                     190                     195
   GCA TAT CCA ACG TTT GAG CAT TTA CAG TTT AAT CCA CCA CCA CTT TTG    2717
   Ala Tyr Pro Thr Phe Glu His Leu Gln Phe Asn Pro Pro Pro Leu Leu
       200                     205                     210
   CTT GGA CAG TCC AGC AGT GAA GAG GAG AAG AAA AAT GAA ACA AAG GGT    2765
   Leu Gly Gln Ser Ser Ser Glu Glu Glu Lys Lys Asn Glu Thr Lys Gly
   215                     220                     225                     230
   TCC ATG CTA AAA CTT ACA AAA CAT CAA GTT GAA ATG TTG AGA AAA AAG    2813
   Ser Met Leu Lys Leu Thr Lys His Gln Val Glu Met Leu Arg Lys Lys
                   235                     240                     245
   GCG AAC CAA GGT AAT CAA GGG CGT AGT TAC ACA CGT TAT GAA GTT GTG    2861
   Ala Asn Gln Gly Asn Gln Gly Arg Ser Tyr Thr Arg Tyr Glu Val Val
               250                     255                     260
   ACT GCA CAT ATA TGG AGA TGT GCA TGC AAG GCA AGA GGT CAT AAA TTT    2909
   Thr Ala His Ile Trp Arg Cys Ala Cys Lys Ala Arg Gly His Lys Phe
           265                     270                     275
   GAG CAG CCT ACT AAT TTA TGC ATT TGT GTT AAC ATA CGC AAT ATA ATG    2957
   Glu Gln Pro Thr Asn Leu Cys Ile Cys Val Asn Ile Arg Asn Ile Met
       280                     285                     290
   CAA CCA CCT TTG CCT AAA TCC TAT TTT GGC AAT GCC ATA GTT GAT GTT    3005
   Gln Pro Pro Leu Pro Lys Ser Tyr Phe Gly Asn Ala Ile Val Asp Val
   295                     300                     305                     310
   ATT GCC AAT GGC GTC TCG GGT GAC ATT ACC TCG AGG CCA TTG GAG TAT    3053
   Ile Ala Asn Gly Val Ser Gly Asp Ile Thr Ser Arg Pro Leu Glu Tyr
                       315                         320                         325
   GTT GCT CGA AGG GTG CGA GCA GCC ATT AAA ATG GTG ACG AGT GAT TAC   3101
   Val Ala Arg Arg Val Arg Ala Ala Ile Lys Met Val Thr Ser Asp Tyr
                   330                        335                        340
   GCA AAC TCG ACG ATT GAT TTC TTA AAA AAC CAG GAG GAT TTG TCA AAA   3149
   Ala Asn Ser Thr Ile Asp Phe Leu Lys Asn Gln Glu Asp Leu Ser Lys
               345                       350                       355
   TAT CAA GAT ATT CAT GCA TTT AGA AGC AAG GAA GGT CCT TTT TAT GGA   3197
   Tyr Gln Asp Ile His Ala Phe Arg Ser Lys Glu Gly Pro Phe Tyr Gly
           360                       365                       370
   AAC CCT AAT CTT GGG GTT ATA AGT TGG ATA AGT TTG CCA TTA TTA GGA   3245
   Asn Pro Asn Leu Gly Val Ile Ser Trp Ile Ser Leu Pro Leu Leu Gly
   375                       380                       385                       390
   TTG GAT TTT GGG TGG GGA AAA GAG ATA CAT ATG AGC CCT GGA ACT CAT   3293
   Leu Asp Phe Gly Trp Gly Lys Glu Ile His Met Ser Pro Gly Thr His
                       395                       400                       405
   GAA TAT GAT GGT GAT TGT GTG ATA CTT CCA GGA AAA GAA GGG GAT GGA   3341
   Glu Tyr Asp Gly Asp Cys Val Ile Leu Pro Gly Lys Glu Gly Asp Gly
                   410                       415                       420
   TCT TTG ACT GTT GCA ATC ATT CTT CAA GCT GTT CAT GTG GAT GCT TTC   3389
   Ser Leu Thr Val Ala Ile Ile Leu Gln Ala Val His Val Asp Ala Phe
               425                       430                       435
   AAG AAC TTC TTC TAT GAA GAA ATT GAA TGT TGAAAAACAT AAGTGTTTTA     3439
   Lys Asn Phe Phe Tyr Glu Glu Ile Glu Cys
           440                       445
   TGAGAAGAAA GGAAACAAAT TAAGAACATG TAGCTTTTCC TAAATTGACA TTGTTAGTCA 3499
   TGGTCTAAGC AAAATAAACT CTTTATCTAC ACATTATTTC AATATATTTT CCTTATTTTC 3559
   TATCAGATTT CTCATATGTT TATTTGATGT TCTTAATTTT ACGAACAATA ATCGGTCATA 3619
   AATGGTTTGA AAATCAATAA CCAAAACTGG AACTATATTG ATTGTTTGGA AGCTAAGCAC 3679
   TTTTTTTCTT CTTTTTTCGC AAAGCAC                                     3706